# EUROPÄISCHE PATENTSCHRIFT

(11) **EP 0 365 913 B1**
(45) Veröffentlichungstag und Bekanntmachung des Hinweises auf die Patenterteilung: **18.11.1993**
(21) Anmeldenummer: 89118835.1
(22) Anmeldetag: 11.10.1989
(51) Int. Cl.: C07D 417/12, A61K 31/505

(54) **Substituierte 2-Aminothiazole**
Substituted 2-aminothiazoles
2-Aminothiazoles substitués

(30) Priorität: 24.10.1988 DE 3836184
(43) Veröffentlichungstag der Anmeldung: 02.05.1990
(73) Patentinhaber: BAYER AG, 51368 Leverkusen (DE)
(72) Erfinder: Ippen, Joachim, Dr., D-5090 Leverkusen 1 (DE); Baasner, Bernd, Dr., D-5090 Leverkusen 3 (DE); Schaller, Klaus, Dr., D-5600 Wuppertal 1 (DE); von Bittera, Miklos, D-5090 Leverkusen 1 (DE)

(56) Entgegenhaltungen:
- EP-A- 0 095 640
- FR-A- 2 042 315

## Beschreibung

Die Erfindung betrifft neue substituierte 2-Aminothiazole, ein Verfahren zu ihrer Herstellung und ihre Verwendung bei der Bekämpfung von Krankheiten, insbesondere Mykosen.

Es ist bekannt, daß bestimmte substituierte Aminothiazole, wie beispielsweise die Verbindung 4-(2,4-Dichlorphenyl)-2-[2-(1,4,5,6-tetrahydropyrimidinyl)-amino]-thiazol-Hydrochlorid oder die Verbindung 4-(4-Chlor-2-methylphenyl)-2-[2-(1,4,5,6-tetrahydropyrimidinyl)-amino]thiazol-Hydrochlorid oder die Verbindung 4-(4-Chlor-2-methoxyphenyl)-2-[2-(1,4,5,6-tetrahydropyrimidinyl)-aminol-thiazol gute antimykotische Eigenschaften besitzen (vgl. z. B. DE- A 32 20 118).

Die Wirksamkeit dieser bekannten Verbindungen ist jedoch nicht in allen Indikationen völlig zufriedenstellend.

Es wurden neue substituierte 2-Aminothiazole der allgemeinen Formel (I), 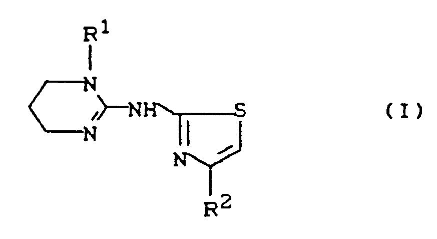
in welcher
- R¹: für Wasserstoff oder für geradkettiges oder verzweigtes Alkyl mit 1 bis 4 Kohlenstoffatomen steht und
- R²: für einfach oder mehrfach, gleich oder verschieden substituiertes Phenyl steht, wobei als Substituenten in Frage kommen: Fluor oder Chlor, geradkettiges oder verzweigtes Alkyl mit 1 bis 6 Kohlenstoffatomen sowie jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Fluor- oder Chloratomen.

sowie deren physiologisch verträgliche Säureadditionssalze,
- mit Ausnahme der Verbindungen 4-(4-Chlorphenyl)-2-[2-(1,4,5,6-tetrahydropyrimidinyl)-amino]-thiazol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydropyrimidinyl)-amino]-thiazol sowie mit Ausnahme der physiologisch verträglichen Säureadditionssalze dieser Verbindungen -

gefunden.

Die Verbindungen der Formel (I) stehen im Gleichgewicht mit den tautomeren Verbindungen der Formeln (Ia) und (Ib), 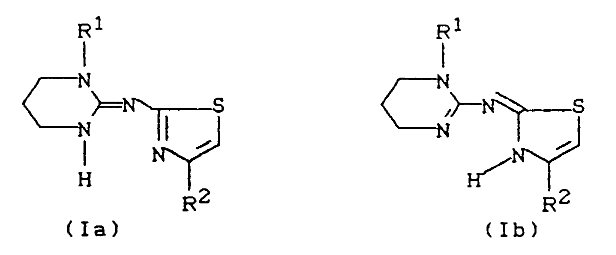
(wobei R¹ und R² jeweils die oben angegebene Bedeutung haben) deren Verwendung ebenfalls erfindungsgemäß beansprucht wird.

Weiterhin wurde gefunden, daß man die vorstehend beschriebenen substituierten 2-Aminothiazole der allgemeinen Formel (I), erhält, wenn man Thioharnstoff-Derivate der Formel (II), 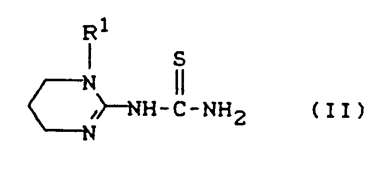
in welcher
- R¹: die oben angegebene Bedeutung hat,
mit Keton-Derivaten der Formel (III), 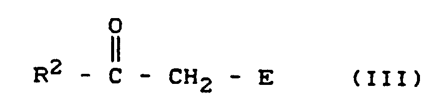
in welcher
- R²: die oben angegebene Bedeutung hat und
- E: für Hydroxy oder Halogen steht,
gegebenenfalls in Gegenwart eines Verdünnungsmittels und gegebenenfalls in Gegenwart eines Reaktionshilfsmittels umsetzt und gegebenenfalls anschließend eine Säure addiert.

Schließlich wurde gefunden, daß die neuen substituierten 2-Aminothiazole der allgemeinen Formel (I) gute antimikrobielle, insbesondere gute antimykotische Eigenschaften besitzen.

Überraschenderweise zeigen die erfindungsgemäßen substituierten 2-Aminothiazole der allgemeinen Formel (I) in bestimmten Indikationen eine erheblich bessere antimykotische Wirksamkeit als die aus dem Stand der Technik bekannten substituierten Aminothiazole, wie beispielsweise die Verbindung 4-(2,4-Dichlorphenyl)-2-[2-(1,4,5,6-tetrahydropyrimidinyl)-amino]-thiazol-Hydrochlorid oder die Verbindung 4-(4-Chlor-2-methylphenyl)-2-[2-(1,4,5,6-tetrahydropyrimidinyl)-amino]-thiazol-Hydrochlorid oder die Verbindung 4-(4-Chlor-2-methoxyphenyl)-2-[2-(1,4,5,6-tetrahydropyrimidinyl)-amino]-thiazol, welche chemisch und wirkungsmäßig naheliegende Verbindungen sind.

Bevorzugt sind Verbindungen der Formel (I), bei welchen
- R¹: für Wasserstoff oder Methyl steht und
- R²: für ein- bis fünffach gleich oder verschieden substituiertes Phenyl steht, wobei als Substituenten in Frage kommen: Fluor, Chlor, geradkettiges oder verzweigtes Alkyl mit 1 bis 4 Kohlenstoffatomen oder jeweils geradkettiges oder verzweigtes Halogenalkyl, Halogenalkoxy, Dioxyhalogenalkylen oder Halogenalkylthio mit jeweils 1 bis 3 Kohlenstoffatomen und 1 bis 7 gleichen oder verschiedenen Fluor- oder Chloratomen,

- mit Ausnahme der Verbindungen 4-(4-Chlorphenyl)-2-[2-(1,4,5,6-tetrahydropyrimidinyl)-amino]-thiazol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydropyrimidinyl)-amino]-thiazol sowie mit Ausnahme der physiologisch verträglichen Säureadditionssalze dieser Verbindungen -.

Bevorzugte erfindungsgemäße Verbindungen sind auch Additionsprodukte aus Säuren und denjenigen substituierten 2-Aminothiazolen der Formel (I), in denen die Substituenten R¹ und R² die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsaure, Maleinsäure, Bernsteinsäure, Fumarsäure, Glutarsäure, Hydroxyglutarsäure, Adipinsäure, Oleinsäure, Malonsäure, Oxalsäure, Weinsäure, Äpfelsäure, Zitronensäure, Benzoesäure, Salicylsäure, Sorbinsäure und Milchsäure, Sulfonsäuren, wie z.B. Methansulfonsäure, p-Chlorbenzolsulfonsäure, p-Toluolsulfonsäure und 1,5-Naphthalindisulfonsäure, Schwefelsäurehalbester wie Schwefelsäuremonomethylester oder Schwefelsäuremonoethylester sowie Saccharin oder Thiosaccharin.

Besonders bevorzugt sind Verbindungen der Formel (I), bei welchen
- R¹: für Wasserstoff steht,
- R²: für ein- bis fünffach, gleich oder verschieden durch Fluor, Chlor, Methyl, Ethyl, Trifluormethyl, Trifluormethoxy, Trifluormethylthio, Dioxydifluormethylen oder Dioxytetrafluorethylen substituierte Phenyl steht,

- mit Ausnahme der Verbindungen 4-(4-Chlorphenyl)-2-[2-(1,4,5,6-tetrahydropyrimidinyl)-amino]-thiazol und 4-(2,4-Dichlorphenyl)-2-[2-(1,4,5,6-tetrahydropyrimidi nyl)-amino]-thiazol und 4-(4-Chlor-2-methylphenyl)-2-[2-(1,4,5,6-tetrahydropyrimidinyl)-amino]-thiazol sowie mit Ausnahme der physiologisch verträglichen Säureadditionssalze dieser Verbindungen -.

Verwendet man beispielsweise N-(1,4,5,6-Tetrahydro-2-pyrimidinyl)-thioharnstoff und ω-Hydroxy-3-trifluormethyl-acetophenon als Ausgangsstoffe, so läßt sich der Reaktionsablauf des Herstellungsverfahrens durch das folgende Formelschema darstellen: 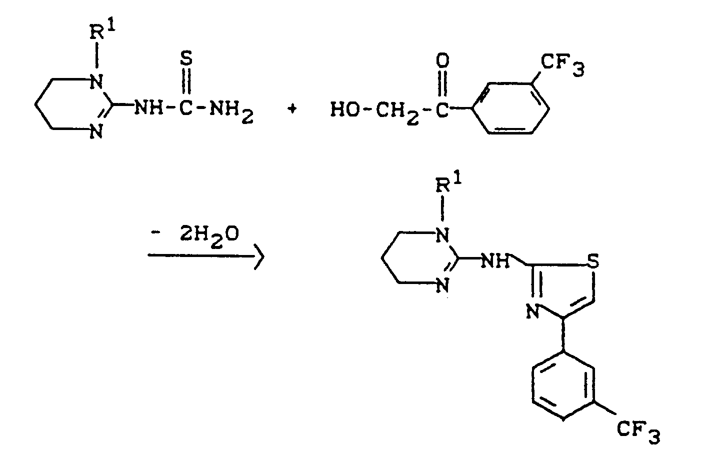
Die zur Durchführung des erfindungsgemäßen Verfahrens als Ausgangsstoffe benötigten Thioharnstoff-Derivate sind durch die Formel (II) allgemein definiert. In dieser Formel (II) steht R¹ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thioharnstoff-Derivate der Formel (II) sind bekannt (vgl. z. B. Arzneim.-Forsch. 35, 573 - 577 [1985] oder DE- A- 32 20 118 bzw. EP-A-95 640) oder erhältlich in Analogie zu bekannten Verfahren (vgl. z. B. Organic Syntheses. Coll. Vol. IV, 502), beispielsweise wenn man Tetrahydropyrimidinylcyanamide der Formel (IV), 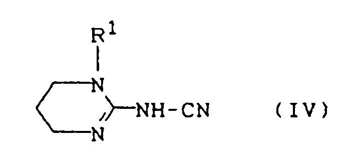
in welcher
- R¹: die oben angegebene Bedeutung hat,
mit Schwefelwasserstoff gegebenenfalls in Gegenwart eines Verdünnungsmittels wie beispielsweise Wasser und gegebenenfalls in Gegenwart eines Reaktionshilfsmittels wie beispielsweise Natriumhydroxid bei Temperaturen zwischen 20 °C und 120 °C umsetzt.

Tetrahydropyrimidinylcyanamide der Formel (IV) sind bekannt (vgl. z. B. DE- A- 22 05 745; DE- A- 22 05 744; J. Org. Chem. 38, 155 - 156 [1973]).

Die zur Durchführung des erfindungsgemäßen Verfahrens weiterhin als Ausgangsstoffe benötigten Keton-Derivate sind durch die Formel (III) allgemein definiert. In dieser Formel (III) steht R² vorzugsweise für diejenigen Reste, die bereits im Zusammenhang mit der Beschreibung der erfindungsgemäßen Stoffe der Formel (I) als bevorzugt für diesen Substituenten genannt wurden. E steht vorzugsweise für Hydroxy, Chlor oder Brom.

Die Keton-Derivate der Formel (III) sind zum Teil bekannt oder in Analogie zu allgemein bekannten Verfahren erhältlich (vgl. z. B. DE-A- 24 45 120; US-A- 37 63 148; US-A- 37 53 997 oder Org. Mass. Spectrom. 18, 601 - 607 [1983]).

Neue Keton-Derivate der allgemeinen Formel (III) sind solche der Formel (IIIa) 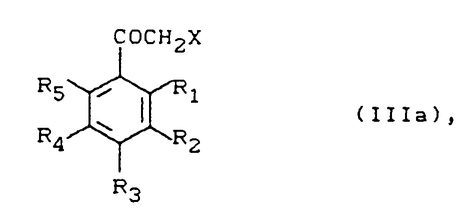
in der
- X: für Wasserstoff, Chlor oder Brom steht und
die Reste R₁ bis R₅ folgende Bedeutung haben,
a) R₁ und R₄ Fluor, R₂ und R₅ Chlor und R₃ CF₃ oder
b) R₁, R₃ und R₄ Fluor, und R₂ und R₅ Wasserstoff oder
c) R₁, R₄ und R₅ Wasserstoff, R₂ Chlor und R₃ CF₃ oder
d) R₁, R₄ und R₅ Wasserstoff, R₂ Chlor und R₃ OCF₃ oder
e) R₁, R₄ und R₅ Wasserstoff und R₂ und R₃ CF₃ oder
f) R₁ Chlor, R₂ CF₃ und R₃, R₄ und R₅ Wasserstoff oder
g) R₁ Chlor, R₂, R₃ und R₄ Wasserstoff und R₅ CF₃ oder
h) R₁ Chlor, R₂, R₄ und R₅ Wasserstoff und R₃ CF₃ bedeuten.

Fluorhaltige Acetophenone der Formel (IIIa) mit X = Wasserstoff, welche die unter c) bis h) angegebenen Bedeutungen der Substituenten haben, sind bevorzugt. Ganz besonders bevorzugt sind fluorhaltige Acetophenone der Formel (IIIa) mit X = Wasserstoff, welche die unter f) bis h) angegebenen Bedeutungen der Substituenten haben, also 2-Chlor-3-trifluormethyl-acetophenon, 2-Chlor-4-trifluormethyl-acetophenon und 2-Chlor-6-trifluormethyl-acetophenon.

Von den an der CH₃-Gruppe halogenierten Acetophenonen der Formel (IIIa) mit X = Chlor oder Brom sind die entsprechenden Verbindungen bevorzugt. Ganz besonders bevorzugt sind dabei 2-Chlor-3-trifluormethyl-phenacylbromid und -chlorid, 2-Chlor-4-trifluormethyl-phenacylbromid und -chlorid und 2-Chlor-6-trifluormethyl-phenacyl-bromid und -chlorid.

Die vorliegende Erfindung betrifft auch ein Verfahren zur Herstellung von fluorhaltigen Acetophenonen der Formel (IIIa), die gegebenenfalls an der CH₃-Gruppe halogeniert sind, das dadurch gekennzeichnet ist, daß man zur Herstellung von Verbindungen der Formel (IIIa) mit X = Wasserstoff ein fluoriertes Benzoesäurederivat der Formel (IIIb) 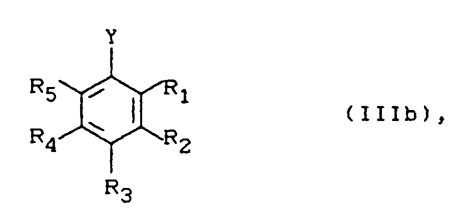
in der
- R₁ bis R₅: die bei Formel (IIIa) angegebene Bedeutung haben und
- Y: für eine Nitrilgruppe oder eine Säurehalogenidgruppe
steht,
mit einer zur Einführung von Methylgruppen befähigten magnesiumorganischen Verbindung umsetzt und dann eine Hydrolyse durchführt und zur Herstellung von Verbindungen der Formel (IIIa) mit X = Chlor oder Brom noch anschließend bei -20 bis +80°C mit einem Chlorierungs- oder Bromierungsmittel umsetzt.

Zum Einsatz in das erfindungsgemäße Verfahren sind solche fluorierten Benzoesäurederivate der Formel (IIIb) bevorzugt, bei denen R₁ bis R₅ die bei Formel (IIIa) unter c) bis h) angegebenen Bedeutungen haben. Besonders bevorzugt sind fluorierte Benzoesäurederivate, bei denen R₁ bis R₅ die bei Formel (IIIa) unter f) bis h) angegebenen Bedeutungen haben.

Soweit in Formel (IIIb) Y für eine Säurehalogenidgruppe steht handelt es sich vorzugsweise um eine Säurefluorid- oder Säurechloridgruppe (COF oder COCl), insbesondere um eine Säurefluoridgruppe (COF).

In Formel (IIIb) steht Y vorzugsweise für eine Nitrilgruppe.

Fluorierte Benzoesäurederivate der Formel (IIIb), bei denen Y für eine Säurehalogenidgruppe steht sind bekannt (siehe z.B. DE-A- 3 621 707).

Fluorierte Benzoesäurederivate der Formel (IIIb), bei denen X für eine Nitrilgruppe steht sind teilweise bekannt und teilweise neu, Neu sind insbesondere fluorierte Benzonitrile der Formel (IIIc) 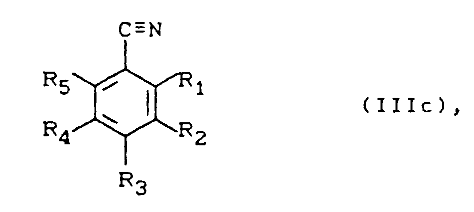
in der
R₁, R₄ und R₅ für Wasserstoff und R₂ und R₃ für CF₃ oder
R₁, R₄ und R₅ für Wasserstoff, R₂ für Chlor und R₃ für OCF₃ oder
R₂, R₃ und R₄ für Wasserstoff, R₁ für Chlor und R₅ für CF₃
stehen.

Die vorliegende Erfindung betrifft deshalb auch solche neuen fluorierten Benzonitrile der Formel (IIIc). Herstellungsmöglichkeiten für die neuen fluorierten Benzonitrile sind in den Beispielen 105a), 106a) und 106b) und 109a) angegeben.

Bei den zur Einführung von Methylgruppen befähigten magnesiumorganischen Verbindungen kann es sich beispielsweise um Methylmagnesiumhalogenide, insbesondere um Methylmagnesiumbromid oder Methylmagnesiumiodid handeln, oder um Ethoxymagnesiummalonester.

Letzterer ist beispielsweise zugänglich, indem man Magnesiumdiethoxylat mit Malonester umsetzt und dabei eine Ethoxygruppe des Magnesiumdiethoxylats durch eine Malonestergruppe ersetzt. Bei Ethoxymagnesiummalonester kann man an das C-Atom im Rest Y des fluorierten Benzoesäurederivates der Formel (IIIb) einen Malonesterrest einführen, der dann bei der Hydrolyse durch Decarboxylierung in eine Methylgruppe übergeht.

Bezogen auf 1 Mol fluoriertes Benzoesäurederivat der Formel (IIIb) kann man beispielsweise 0,8 bis 3 Mol der jeweiligen magnesiumorganischen Verbindung einsetzen. Vorzugsweise beträgt diese Menge 1 bis 1,5 Mol. Die magnesiumorganische Verbindung kommt im allgemeinen in gelöster Form zum Einsatz. Geeignete Lösungsmittel sind beispielsweise Ether, insbesondere Diethylether und Tetrahydrofuran. Im allgemeinen stellt man separat eine Lösung der jeweiligen magnesiumorganischen Verbindung her und fügt diese der Verbindung der Formel (IIIb) zu, die ebenfalls in gelöster Form vorliegen kann. Beim Einsatz von Methylmagnesiumhalogeniden kann es vorteilhaft sein eine kleine Menge Katalysator hinzuzufügen, beispielsweise ein Kupfer- oder Eisensalz.

Die Umsetzung des Benzoesäurederivates der Formel (IIIb) mit der magnesiumorganischen Verbindung kann in einem weiten Temperaturbereich durchgeführt werden, beispielsweise zwischen -60 und +100°C. Die nach dieser Umsetzung durchzuführende Hydrolyse kann z.B. durch Eingießen in oder Zufügen von Wasser und mehrstündiges Halten bei einer Temperatur im Bereich von -10 bis +40°C bewerkstelligt werden. Vorzugsweise setzt man Säure zu, beispielsweise Essig-, Salz- oder Schwefelsäure. Die Durchführung der Hydrolyse in Gegenwart einer starken Säure ist insbesondere dann vorteilhaft, wenn als magnesiumorganische Verbindung Ethoxymagnesiummalonester eingesetzt worden ist.

Das nach der Hydrolyse vorliegende Reaktionsgemisch kann man beispielsweise aufarbeiten indem man die organische Phase daraus abtrennt und diese fraktioniert destilliert.

Durch die Umsetzung eines Benzosäurederivats der Formel (IIIb) mit einer zur Einführung von Methylgruppen befähigten magnesium-organischen Verbindung mit nachfolgender Hydrolyse erhält man Fluor enthaltende Acetophenone der Formel (IIIa) mit X = Wasserstoff. Aus diesen kann man an der CH₃-Gruppe halogenierte Acetophenone der Formel (IIIa) mit X = Chlor oder Brom erhalten, wenn man sie bei -20 bis +80°C mit einem Chlorierungs-oder Bromierungsmittel umsetzt. Ein geeignetes Chlorierungsmittel ist beispielsweise Sulfurylchlorid (SO₂Cl₂); als Bromierungsmittel ist beispielsweise elementares Brom geeignet.

Im allgemeinen setzt man das Chlorierungs- oder Bromierungsmittel in der stöchiometrisch erforderlichen Menge oder im Überschuß ein, beispielsweise 1 bis 1,2 Mol pro Mol Edukt. Geeignete Reaktionstemperaturen sind solche im Bereich -20 bis +80°C, insbesondere solche von 0 bis 40°C.

Man kann die Chlorierung bzw. Bromierung in Anwesenheit oder Abwesenheit oder Lösungsmitteln durchführen. Vorzugsweise arbeitet man in Gegenwart inerter organischer Lösungsmittel, beispielsweise Methylenchlorid oder Eisessig. Ebenso ist es nicht zwingend nötig, aber im allgemeinen vorteilhaft, in Gegenwart katalytischer Mengen einer starken, konzentrierten Mineralsäure zu arbeiten. Beispielsweise sind hier Schwefelsäure oder Salzsäure geeignet.

Das Ende der Chlorierung bzw. Bromierung ist am Aufhören der Gasentwicklung (Chlor- oder Bromwasserstoff) zu erkennen. Man kann dann das Reaktionsgemisch beispielsweise so aufarbeiten, daß man es mit Wasser oder Eiswasser vermischt, mit einem organischen Lösungsmittel ausschüttelt, den organischen Extrakt einengt und den Rückstand destilliert. Gegebenenfalls kann eine weitere Reinigung durchgeführt werden, z.B. durch Umkristallisation, Destillation oder Chromatographie.

Die neuen, Fluor enthaltenden Acetophenone der Formel (IIIa) mit X = Chlor oder Brom lassen sich durch Umsetzung mit einem Thioharnstoff-Derivat der Formel (II) 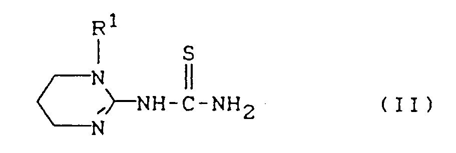
in substituierte Aminothiazole des Typs 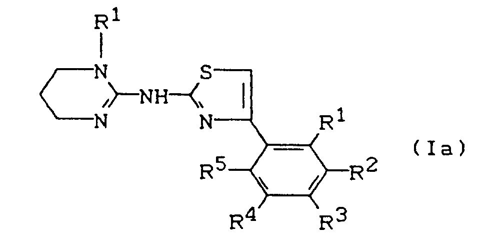
überführen,
bei denen es sich um Verbindungen handelt, die eine gute Wirksamkeit gegen human- und tierpathogene Pilze besitzen.

Es ist als überraschend anzusehen, daß die erfindungsgemäßen fluorierten Acetophenone der Formel (IIIa) auf die beschriebene Weise in guten Ausbeuten zugänglich sind, denn außer der gewünschten Reaktion waren Reaktionen mit den aktivierten, am Kern gebundenen Halogenatomen und Hydrolyse der am Kern gebundenen CF₃-Gruppen zu erwarten.

Als Verdünnungsmittel zur Durchführung des erfindungsgemäßen Verfahrens zur Herstellung der erfindungsgemäßen 2-Aminothiazole kommen inerte organische oder anorganische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid, Alkohole wie Methanol, Ethanol oder Propanol, Basen wie Pyridin, gegebenenfalls durch deren Gemische mit Wasser oder reines Wasser.

Das erfindungsgemäße Verfahren wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, Ammoniak sowie primäre, sekundäre oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem großen Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens setzt man pro Mol an Thioharnstoff-Derivat der Formel (II) im allgemeinen 1.0 bis 1.5 Mol, vorzugsweise 1.0 bis 1.2 Mol an Keton-Derivat der Formel (III) und gegebenenfalls 1.0 bis 1.5 Mol, vorzugsweise 1.0 bis 1.2 Mol an Reaktionshilfsmittel ein.

Die Reaktionsdurchführung, Aufarbeitung und Isolierung der Reaktionsprodukte erfolgt nach allgemein üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können neben den oder die Wirkstoffe die üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

### Herstellungsbeispiele

### Beispiel 1

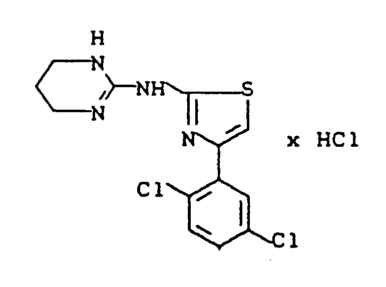

Zu 22,35 g (0,1 Mol) 2,5-Dichlorphenacylchlorid (vgl. z. B. Pharmazie 31, 351 - 354 [1976]; NL 68/7990) in 100 ml Aceton gibt man 15,8 g (0,1 Mol) N-(1,4,5,6-Tetrahydro-2-pyrimidinyl)-thioharnstoff, erhitzt 2 Stunden auf Rückflußtemperatur, kühlt ab auf Raumtemperatur, saugt ausgefallenes Produkt ab, wäscht mit Aceton nach und trocknet.

Man erhält 34 g (94 % der Theorie) an 4-(2,5-Dichlorphenyl)-2-[N-(1,4,5,6-tetrahydro-2-pyrimidinyl)-amino]-thiazol-Hydrochlorid vom Schmelzpunkt 247 °C - 248 °C.

### Beispiel 2

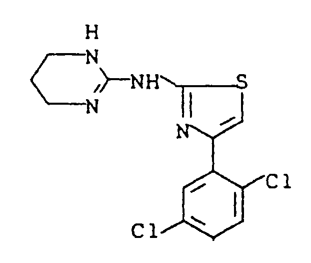

18,18 g (0,05 Mol) 4-(2,5-Dichlorphenyl)-2-[N-(1,4,5,6-tetrahydro-2-pyrimidinyl)-amino]-thiazol-Hydrochlorid werden mit 300 ml 1N-Natronlauge 30 Minuten bei Raumtemperatur gerührt; der so erhältliche Feststoff wird abgesaugt, mit Wasser neutral gewaschen und getrocknet.

Man erhält 34 g (91 % der Theorie) an 4-(2,5-Dichlorphenyl)-2-[N-(1,4,5,6-tetrahydro-2-pyrimidinyl)-amino]-thiazol vom Schmelzpunkt 188 °C - 189 °C.

In entsprechender Weise und gemäß den allgemeinen Angaben zur Herstellung erhält man die folgenden substituierten 2-Aminothiazole der allgemeinen Formel (I):

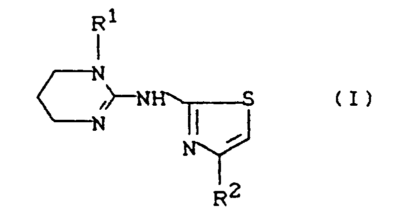

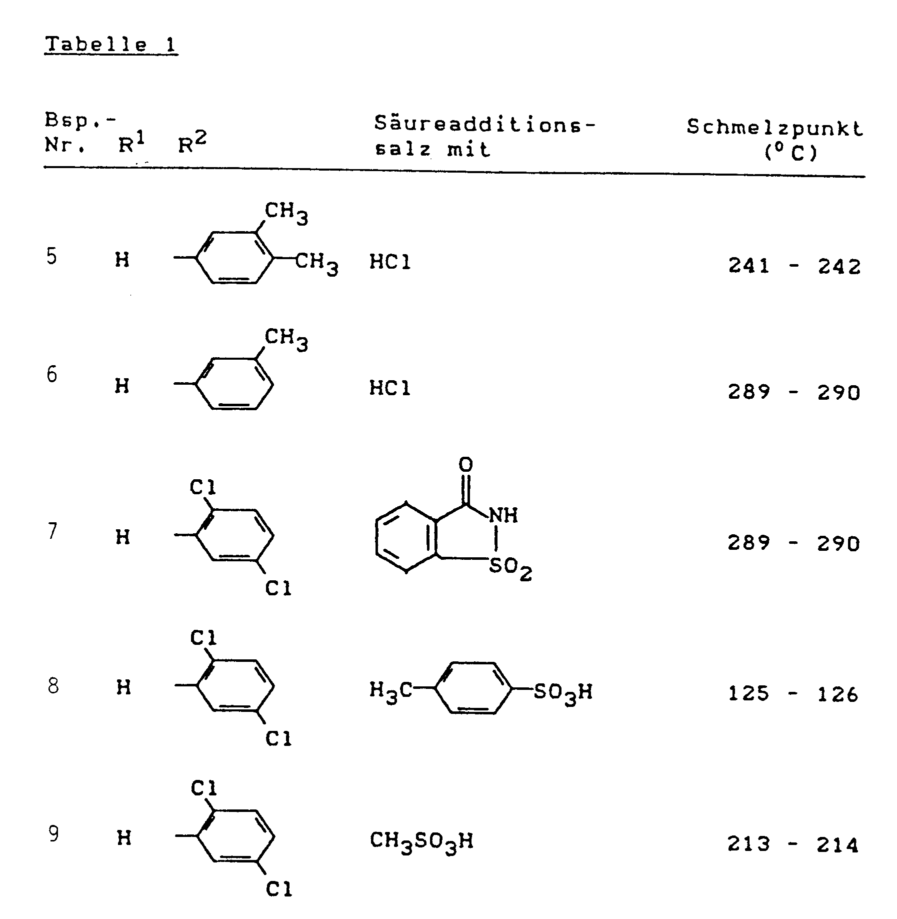

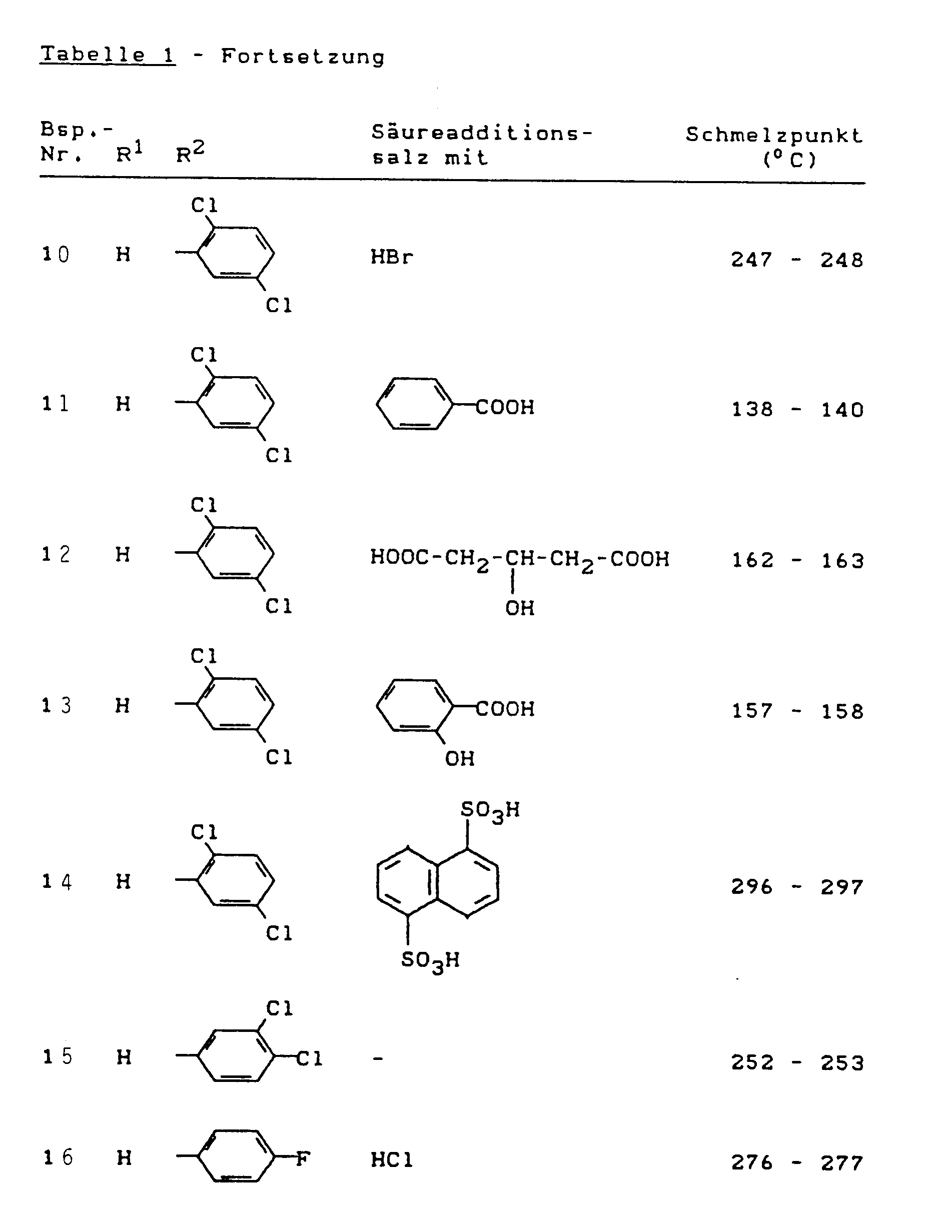

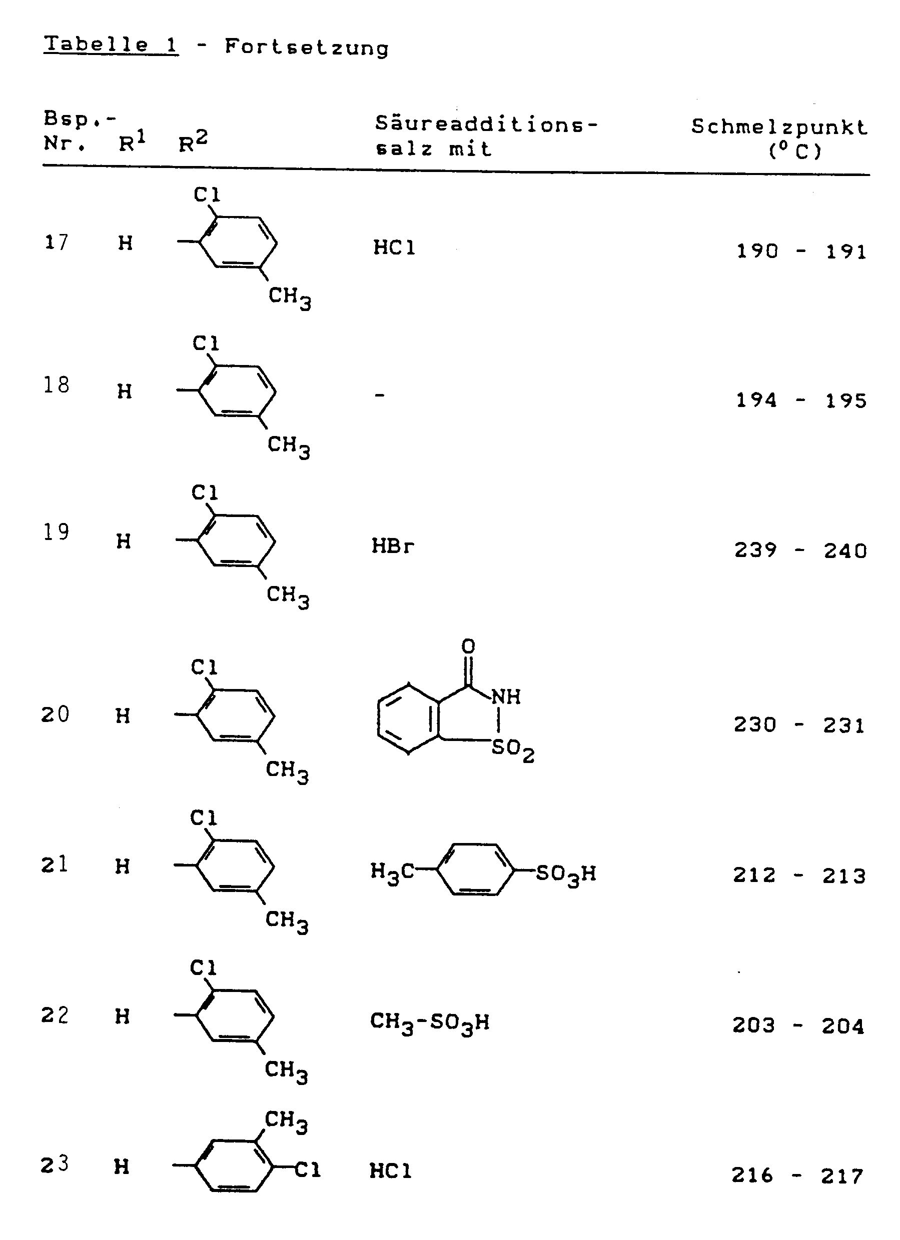

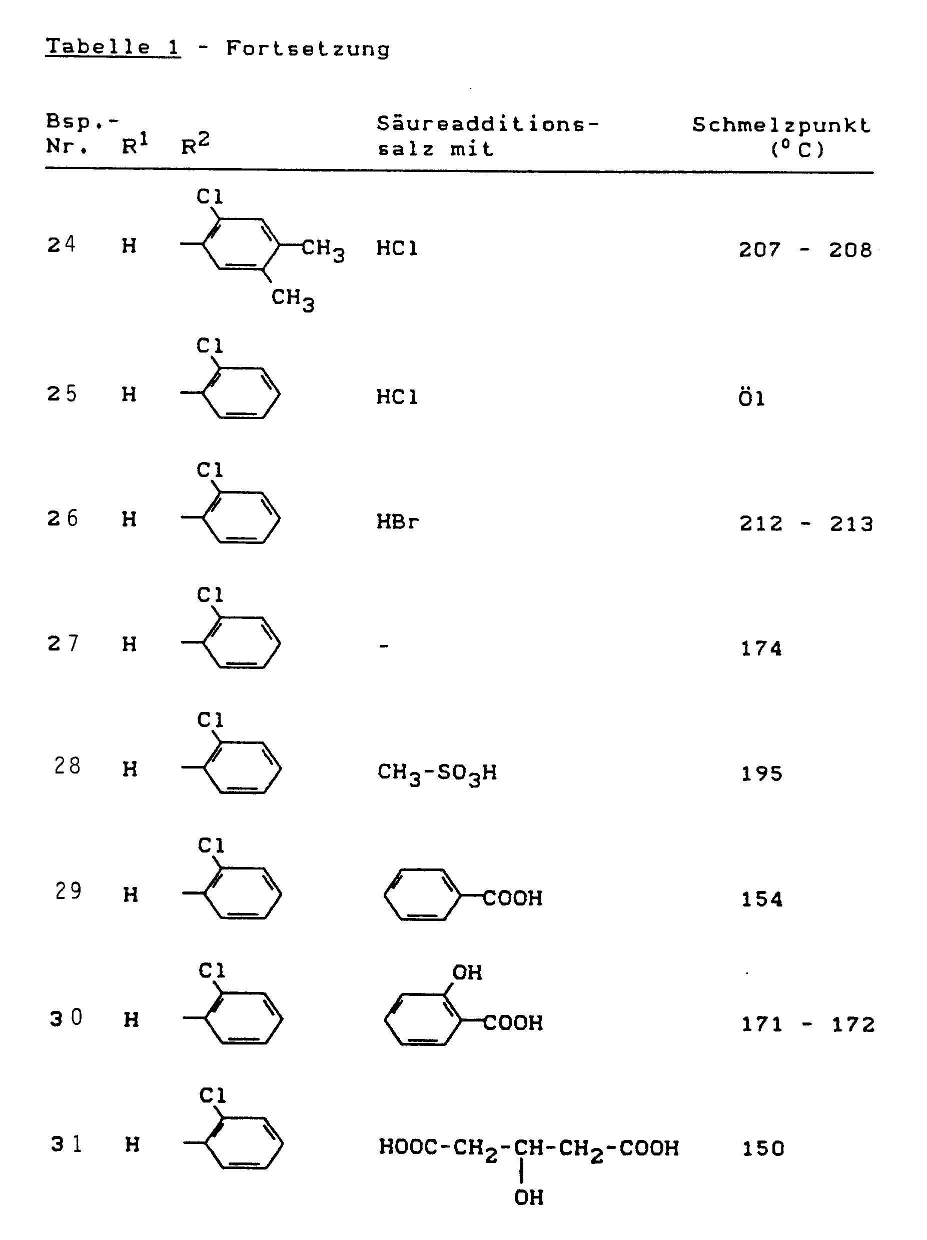

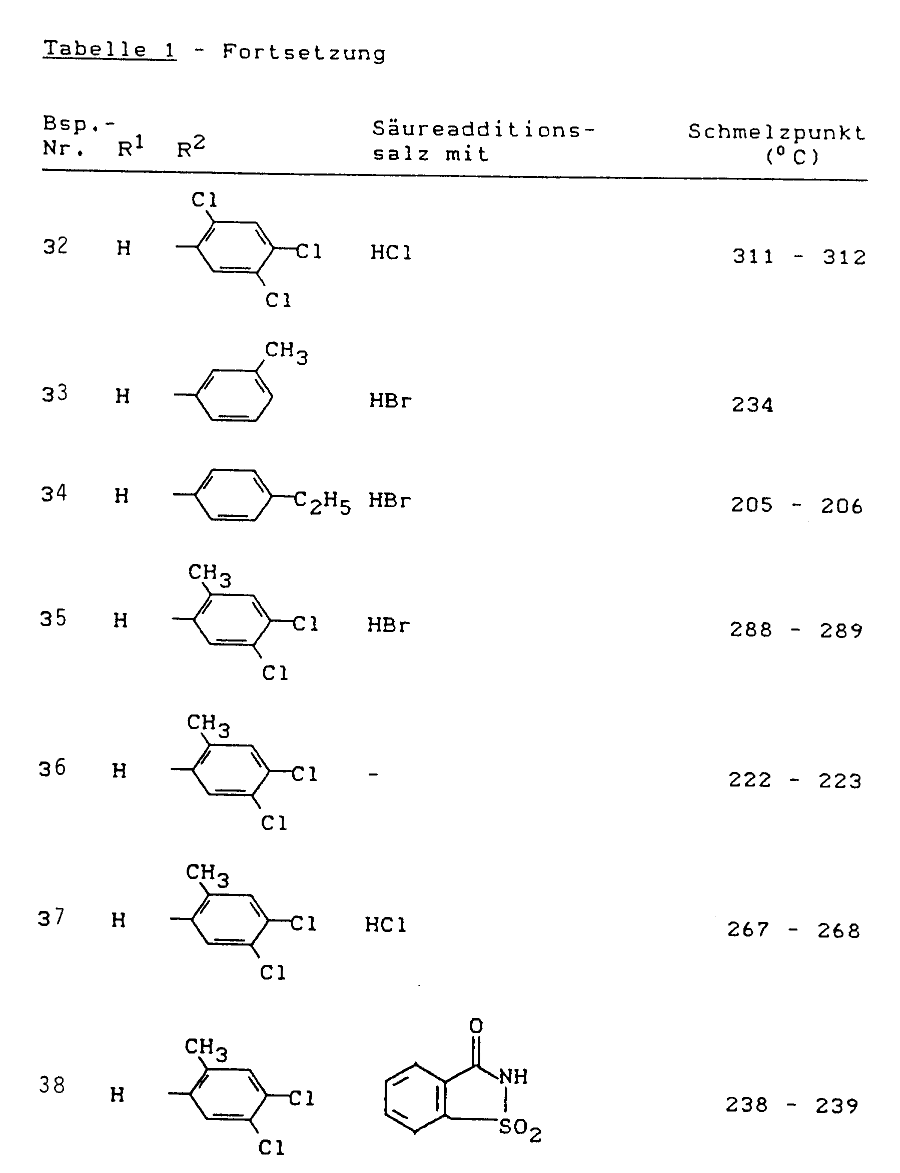

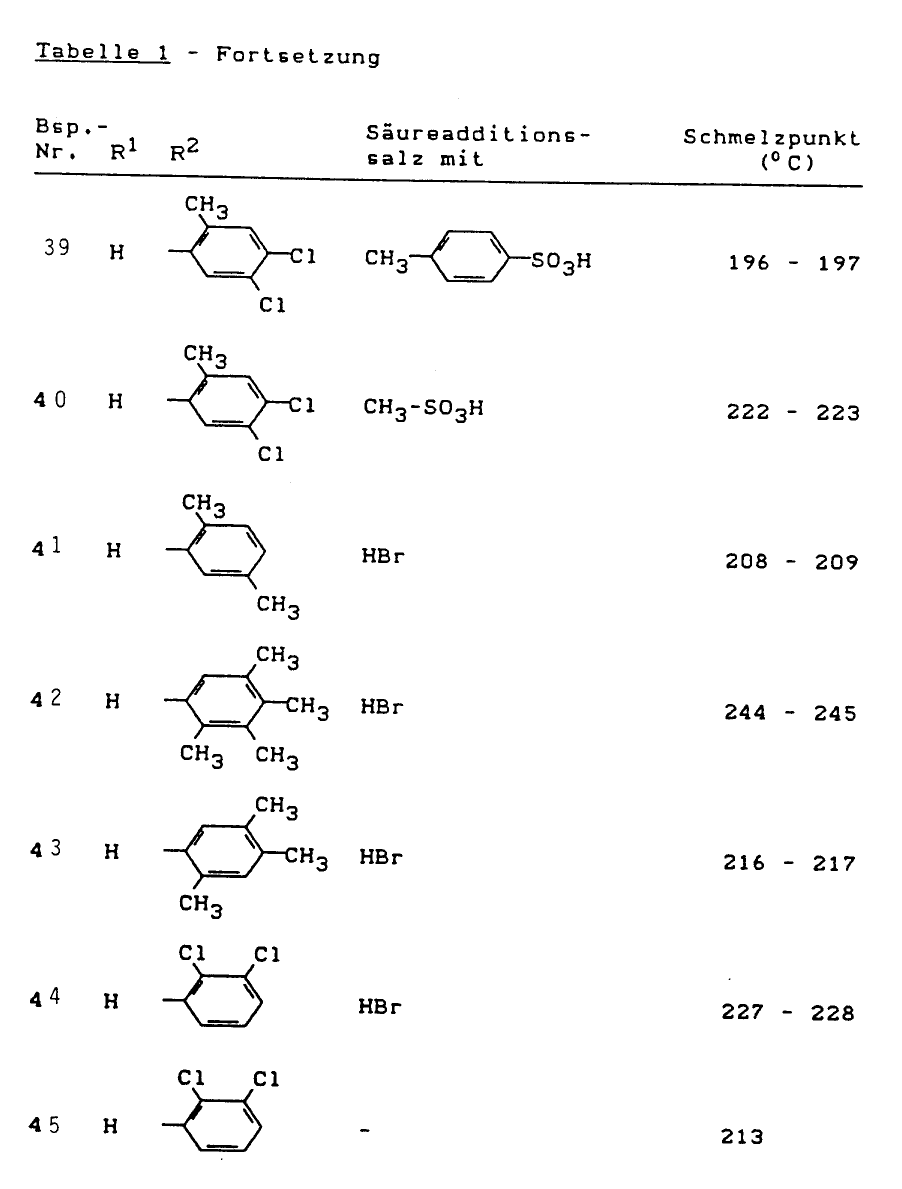

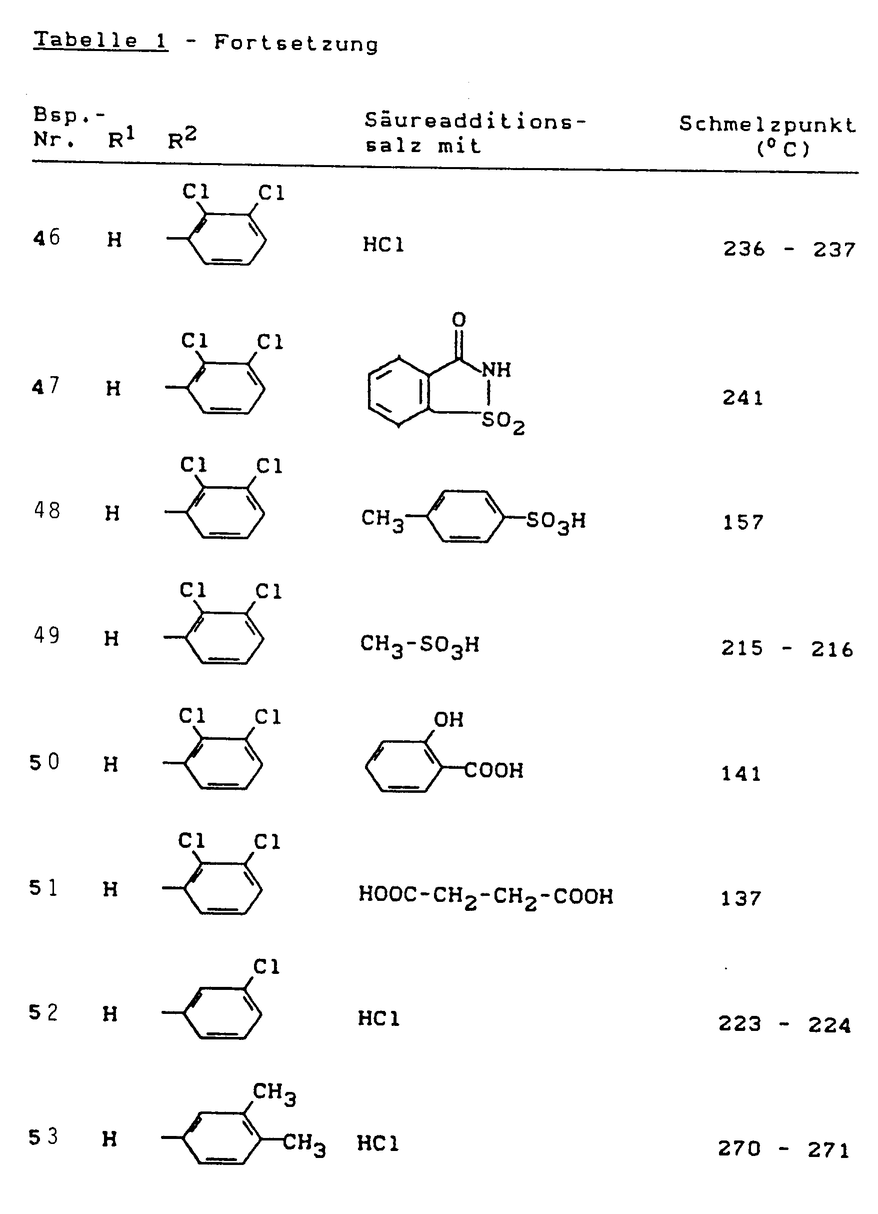

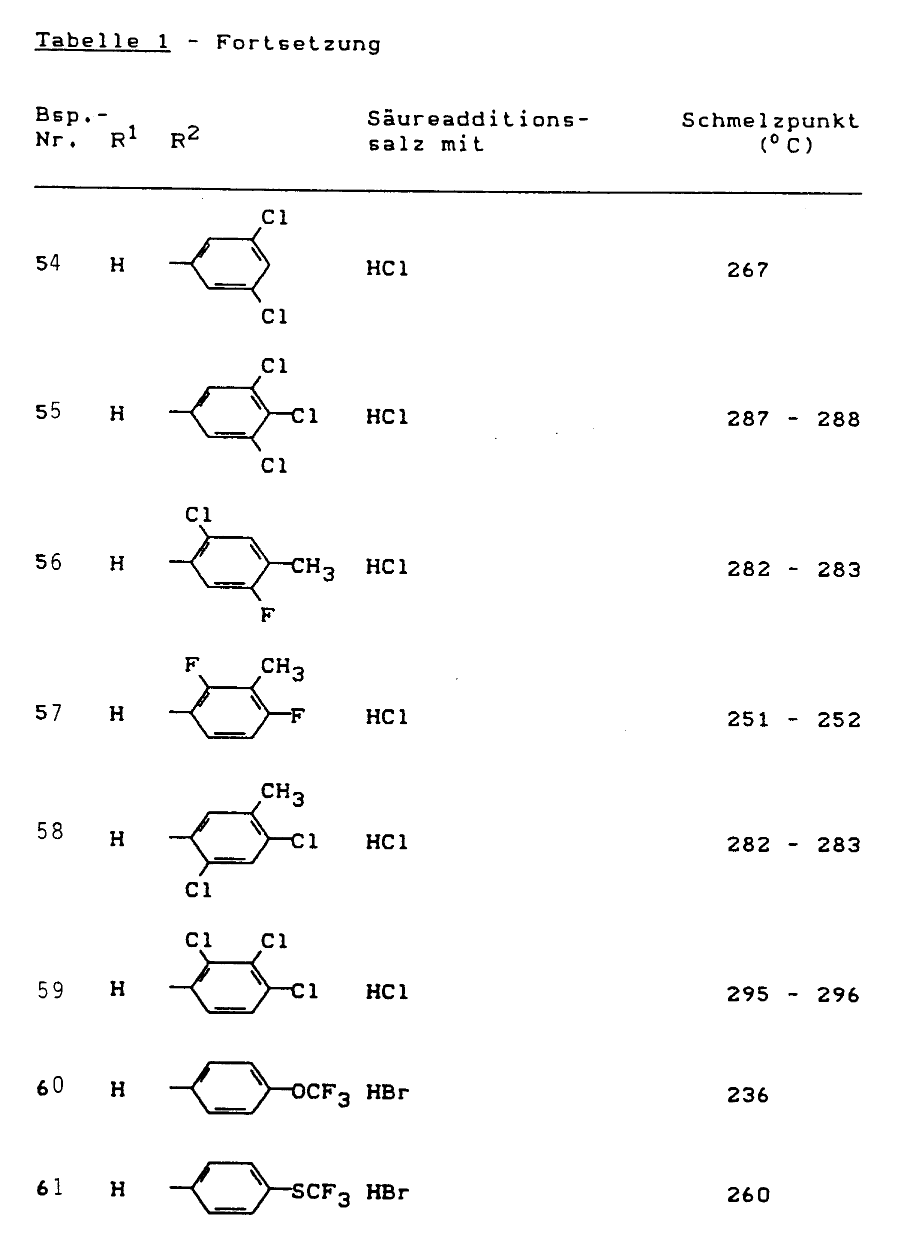

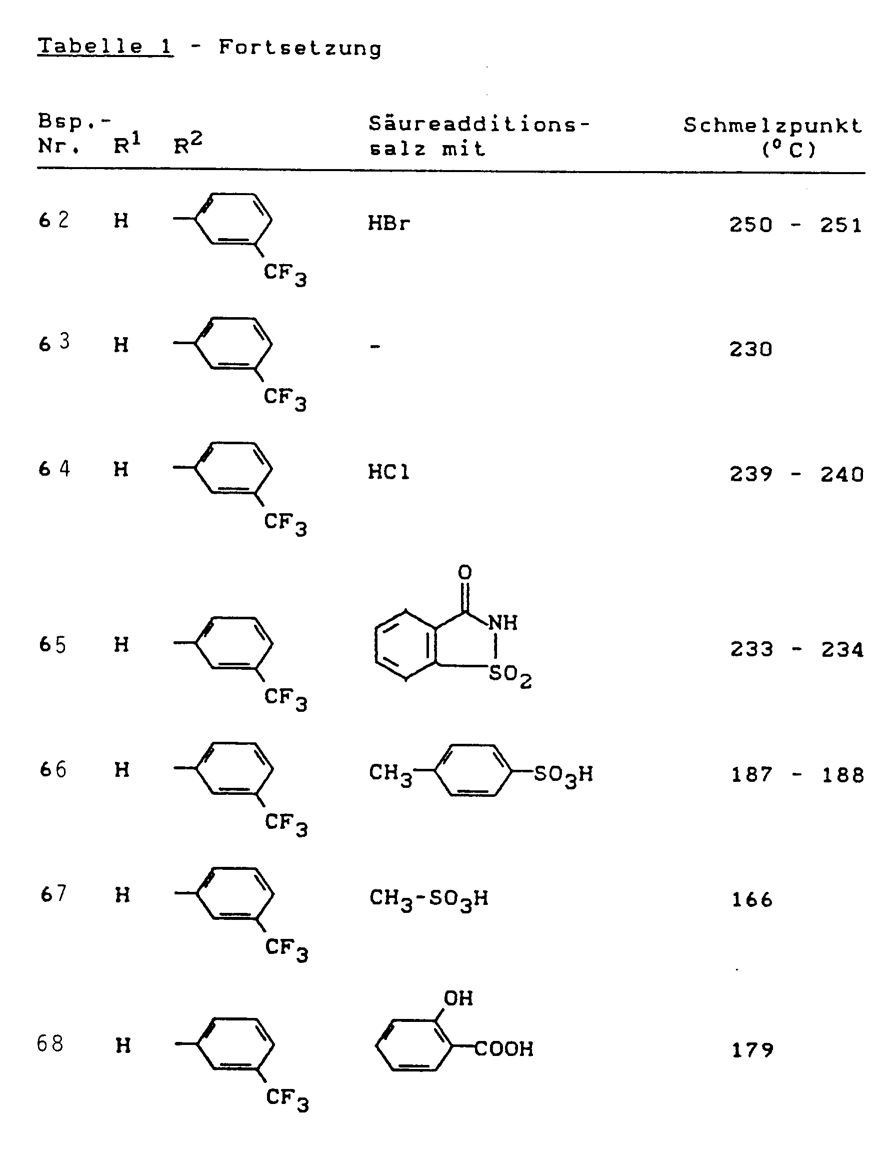

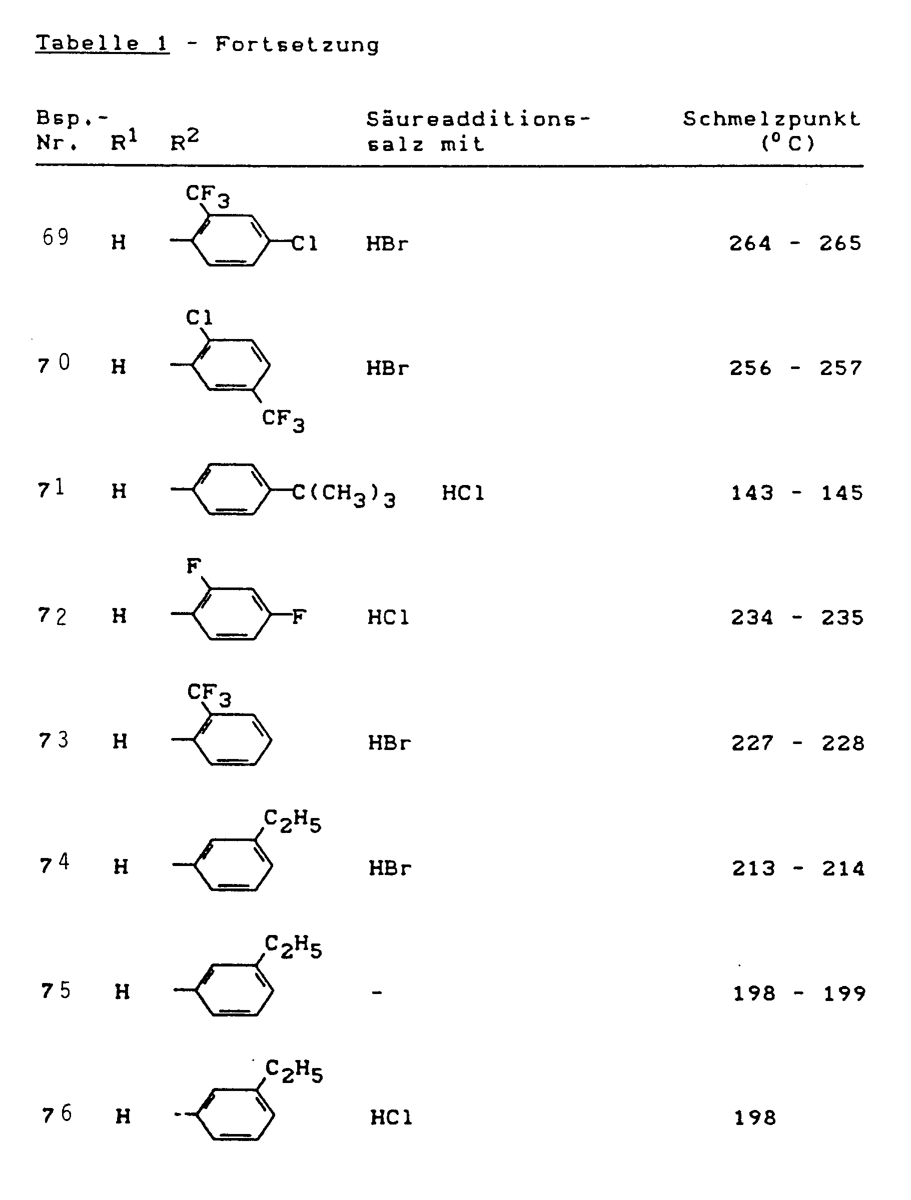

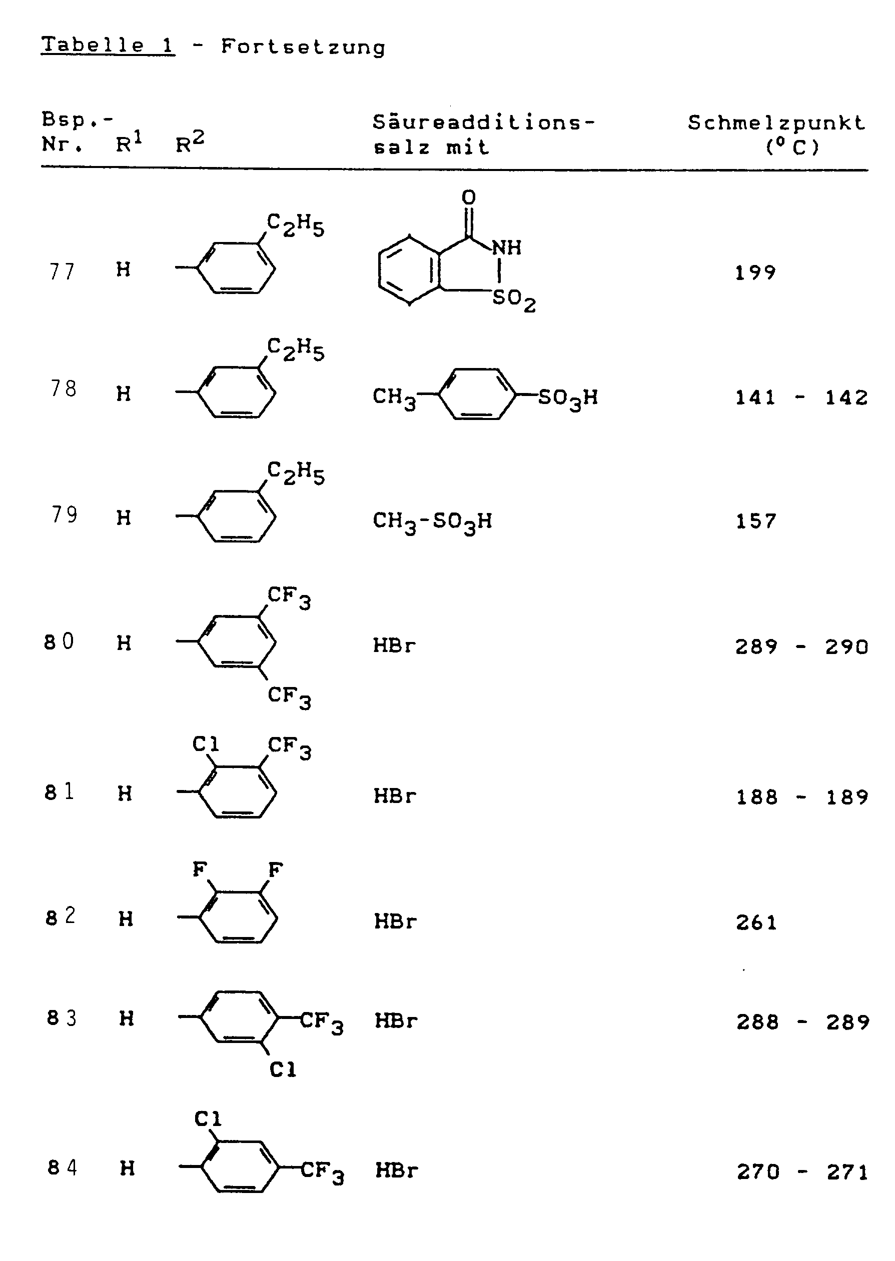

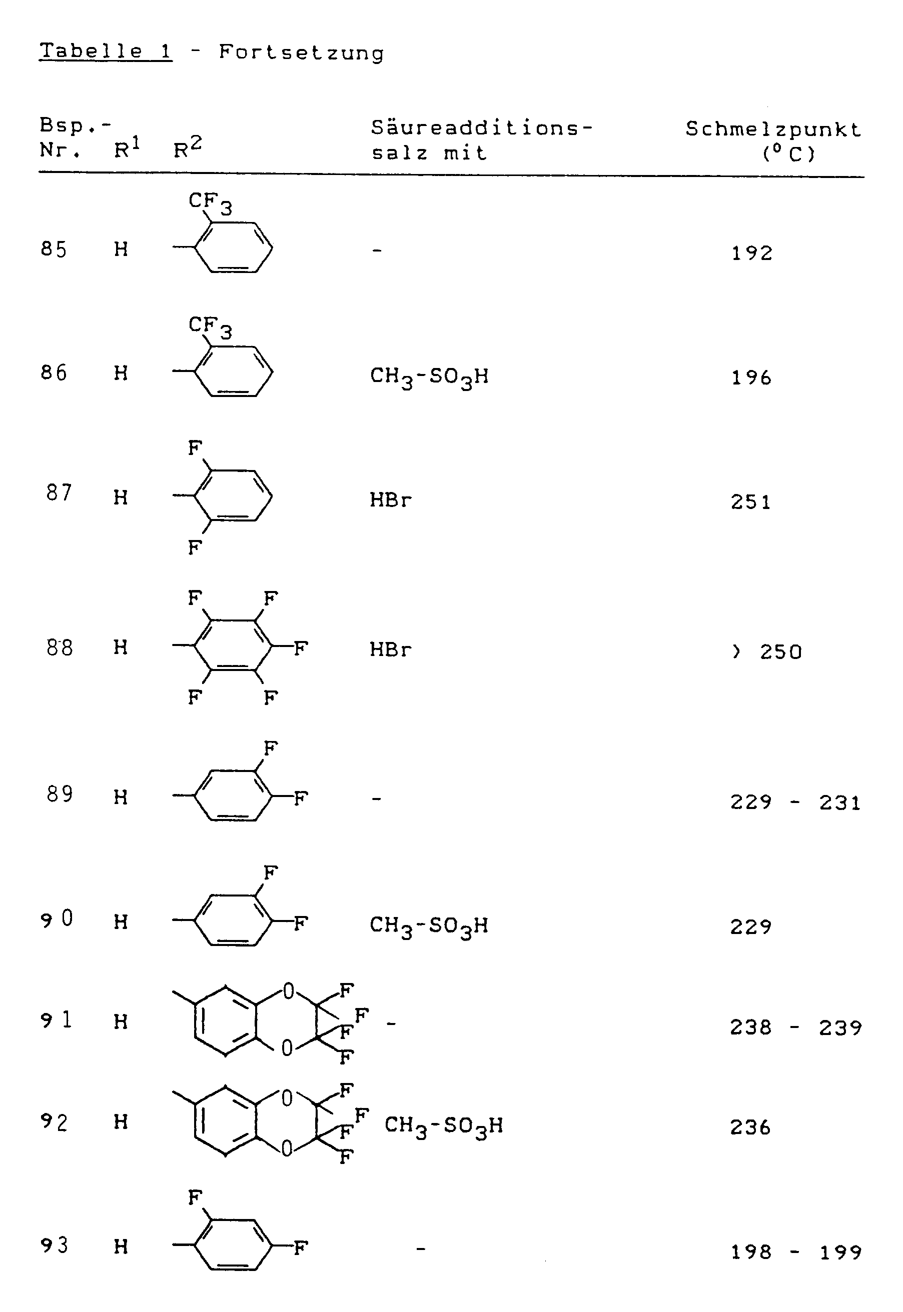

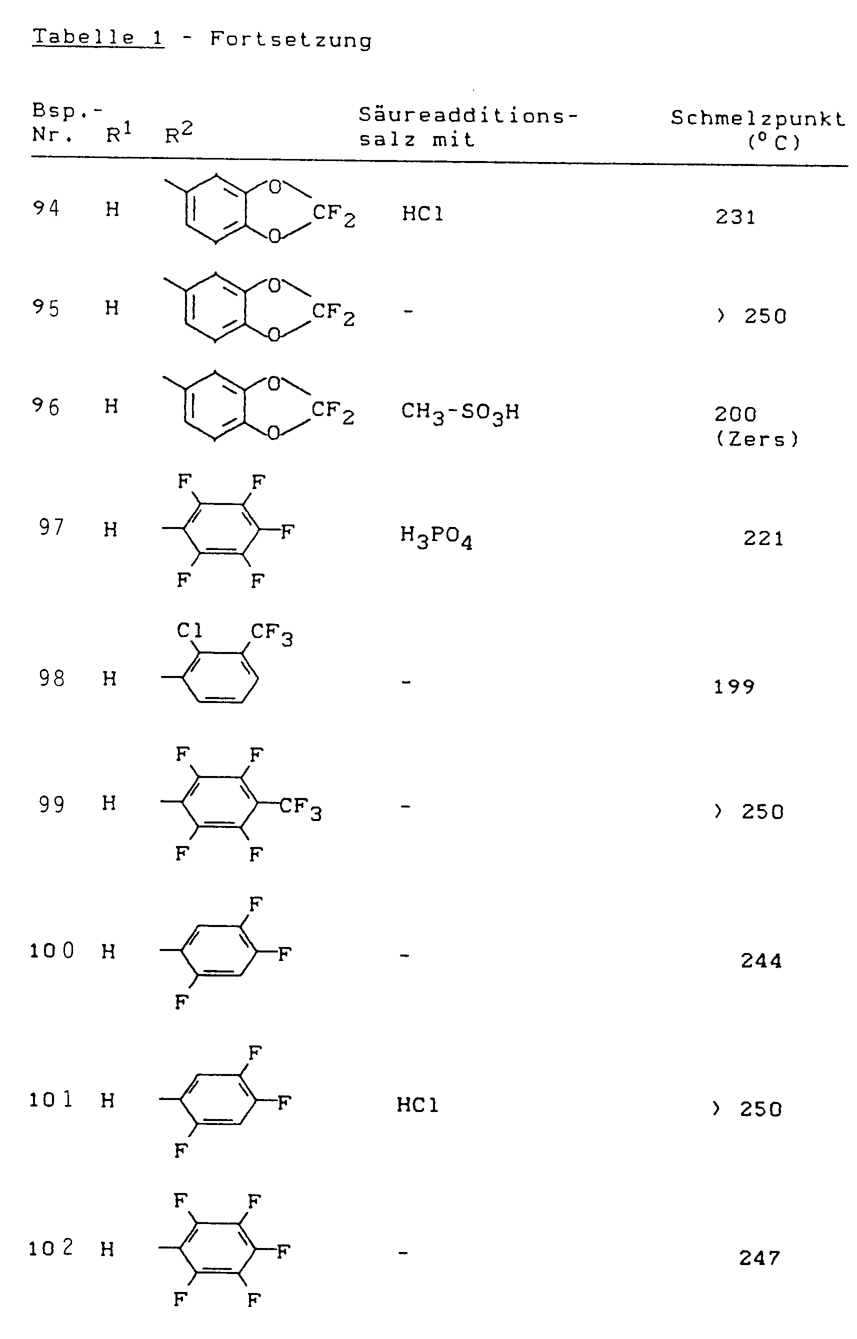

### Zwischenprodukte

### Beispiel 103

### a) 3,4-Bistrifluormethyl-benzonitril

229 g (1 Mol) 3,4-Bistrifluormethyl-anilin wurden in einer Lösung von 350 g konzentrierter Schwefelsäure in 1,25 l Wasser mit 70 g Natriumnitrit in 140 ml Wasser diazotiert. Nachdem die Diazoniumsalzlösung nitritfrei war wurde sie in eine auf 100°C erwärmte Lösung bestehend aus 550 ml Wasser, 210 g Natriumcyanid, 10 g Kupfer(I)-cyanid, 500 g Natriumhydrogencarbonat und 9 g Nickelsulfat x 7 H₂O getropft. Aus dem Reaktionsgemisch wurde das Produkt durch Wasserdampfdestillation isoliert und anschließend nochmals destilliert. Es wurden 152 g Produkt mit einem Siedepunkt bei 14 mbar von 85°C und einem Schmelzpunkt von 71 bis 72°C erhalten. Das entspricht einer Ausbeute von 63 % der Theorie.

### b) 3,4-Bistrifluormethyl-acetophenon

119,5 g (0,5 Mol) 3,4-Bistrifluormethyl-benzonitril wurden mit 166 g (1 Mol) Methylmagnesiumiodid in 750 ml Benzol 3 Stunden am Rückfluß gekocht. Nach dem Abkühlen auf 0° wurden 500 ml 6 n wäßrige Salzsäure zufließen gelassen und weitere 6 Stunden am Rückfluß gekocht. Danach wurde das Gemisch abgekühlt, die organische Phase abgetrennt und destilliert. Es wurden 123 g Produkt mit einem Siedepunkt bei 0,3 mbar von 71 bis 73°C erhalten. Das entspricht einer Ausbeute von 48 % der Theorie.

### Beispiel 104

### a) 3-Chlor-4-trifluormethoxy-benzamid

242,5 g (1 Mol) 3-Chlor-4-trifluormethoxy-benzoylfluorid wurden in 500 ml 25 gew.-%ige wäßrige Ammoniaklösung unter Eiskühlung eintropfen gelassen, danach 30 Minuten nachgerührt und der ausgefallene Niederschlag abgesaugt. Es wurden 227 g Produkt mit einem Schmelzpunkt von 98°C erhalten. Das entspricht einer Rohausbeute von 95 % der Theorie.

### b) 3-Chlor-4-trifluormethoxy-benzonitril

239,5 g (1 Mol) 3-Chlor-4-trifluormethoxy-benzamid wurden mit 750 ml SOCl₂ versetzt und die Mischung langsam (entsprechend der Gasentwicklung) bis auf 85°C erwärmt. Anschließend wurde das Gemisch fraktioniert destilliert und 189 g Produkt mit einem Siedepunkt bei 13 mbar von 96°C und einem Schmelzpunkt von 38 bis 40°C erhalten. Das entspricht einer Ausbeute von 85 % der Theorie.

### c) 3-Chlor-4-trifluormethoxy-acetophenon

Analog Beispiel 103 b) wurden 221,5 g (1 Mol) 3-Chlor-4-trifluormethoxy-benzonitril mit Methylmagnesiumiodid umgesetzt und das Reaktionsgemisch entsprechend aufgearbeitet. Es wurden 105,4 g Produkt mit einem Siedepunkt bei 0,1 mbar von 98 bis 99°C erhalten. Das entspricht einer Ausbeute von 44 % der Theorie.

### Beispiel 105

### 3-Chlor-4-trifluormethyl-acetophenon

226 g (1 Mol) 3-Chlor-4-trifluormethyl-benzoylfluorid wurden in 500 ml Diethylether vorgelegt und nach Zusatz von 3 g FeCl₃ eine aus 95 g (1 Mol) Methylbromid und 24,3 g Magnesium in 250 ml Diethylether hergestellte Grignard-Lösung bei -60°C Innentemperatur innerhalb von 4 Stunden zugetropft. Es wurde noch 24 Stunden bei -60°C gehalten, dann auf 25°C erwärmt. Danach wurde das Reaktionsgemisch in Wasser geschüttet, die organische Phase abgetrennt und fraktioniert destilliert. Es wurden 47,5 g Produkt mit einem Siedepunkt bei 0,2 mbar von 84 bis 87°C erhalten. Das entspricht einer Ausbeute von 20 % der Theorie.

### Beispiel 106

### 2-Chlor-4-trifluormethyl-acetophenon

81 g (0,358 Mol) 2-Chlor-4-trifluormethyl-benzoylchlorid wurden analog Beispiel 3 mit Methylmagnesiumbromid umgesetzt und das Reaktionsgemisch entsprechend aufgearbeitet. Es wurden 35,2 g Produkt mit einem Siedepunkt bei 10 mbar von 80 bis 81°C erhalten. Das entspricht einer Ausbeute von 44 % der Theorie.

### Beispiel 107

### a) 2-Chlor-6-trifluormethyl-benzonitril

255 g (1 Mol) 2-Chlor-6-trichlormethyl-benzonitril und 250 g wasserfreier Fluorwasserstoff wurden in einem Autoklaven 4 Stunden lang auf 140°C erwärmt. Der entstehende Chlorwasserstoff wurde bei 25 bar kontinuierlich entweichen gelassen. Danach wurde überschüssiger Fluorwasserstoff abgezogen, der Reaktionsrückstand destilliert, das Destillat im Siedebereich von 80 bis 142°C bei 15 mbar aufgefangen (180 g), dieses mit 55 g Antimontrifluorid versetzt und auf 90°C erwärmt. In die 90°C warme Reaktionsmischung wurde eine kleine Menge Chlor eingeleitet um das Antimontrifluorid zu aktivieren. Danach wurde noch 1 Stunde auf 135°C erwärmt. Zur Aufarbeitung wurde das Reaktionsgemisch in Wasser geschüttet, die organische Phase abgetrennt und destilliert. Es wurden 138 g Produkt mit einem Siedepunkt bei 13 mbar von 112 bis 113°C und einem Schmelzpunkt von 45 bis 47°C erhalten. Das entspricht einer Ausbeute von 67 % der Theorie.

### b) 2-Chlor-6-trifluormethyl-acetophenon

154 g (0,75 Mol) 2-Chlor-6-trifluormethyl-benzonitril wurden in 375 ml Diethylether vorgelegt und nach Zusatz von 1 g CuCl eine aus 24,3 g Magnesium und 95 g (1 Mol) Methylbromid in 250 ml Diethylether hergestellte Grignard-Lösung bei 28 bis 30°C innerhalb von 3 Stunden zugetropft. Es wurde noch 5 Stunden bei 28 bis 30°C nachgerührt. Dann wurde das Reaktionsgemisch in Wasser gegossen, die organische Phase abgetrennt und destilliert. Es wurden 114 g Produkt mit einem Siedepunkt bei 0,3 mbar von 65 bis 66°C erhalten. Das entspricht einer Ausbeute von 68 % der Theorie.

### Beispiel 108

### 2-Chlor-3-trifluormethyl-acetophenon

193 g (0,94 Mol) 2-Chlor-3-trifluormethyl-benzonitril wurden analog Beispiel 103 b) mit Methylmagnesiumbromid umgesetzt und das Reaktionsgemisch entsprechend aufgearbeitet. Es wurden 94 g Produkt mit einem Siedepunkt bei 8 mbar von 90 bis 91°C erhalten. Das entspricht einer Ausbeute von 45 % der Theorie.

### Beispiel 109

### 2,4,5-Trifluor-acetophenon

194,5 g (1 Mol) 2,4,5-Trifluor-benzoylchlorid wurden in 100 ml Diethylether vorgelegt und zum Sieden am Rückfluß erwärmt. Dann wurden 1,1 Mol Ethoxymagnesiummalonester gelöst in 100 ml Ethanol und 125 ml Diethylether innerhalb von 30 Minuten zutropfen gelassen und 1 Stunde bei Rückfluß nachgerührt. Nach dem Abkühlen wurde das Reaktionsgemisch in 500 ml Eiswasser eingerührt, mit konzentrierter Schwefelsäure auf einen pH-Wert von 1 eingestellt und das organische Material (345 g) abgetrennt. Dieses organische Material wurde in 300 ml Essigsäure gelöst und nach dem Zusatz von 37,5 ml konzentrierter Schwefelsäure bis zum Ende der CO₂-Entwicklung am Rückfluß gekocht, was 6 Stunden dauerte. Danach wurde das Reaktionsgemisch abgekühlt, auf Wasser gegossen, die organische Phase abgetrennt und destilliert. Es wurden 83 g Produkt mit einem Siedepunkt bei 10 mbar von 63 bis 64°C erhalten. Das entspricht einer Ausbeute von 47 % der Theorie.

### Beispiel 110

### 2,3,5,6-Tetrafluor-4-trifluormethyl-acetophenon

364 g (1 Mol) 2,3,5,6-Tetrafluor-4-trifluormethylbenzoylfluorid wurden analog Beispiel 105 mit Ethoxymagnesiummalonester umgesetzt und das Reaktionsgemisch entsprechend aufgearbeitet. Es wurden 170 g Produkt mit einem Siedepunkt bei 60 mbar von 98 bis 100°C erhalten. Das entspricht einer Ausbeute von 65 % der Theorie.

### Beispiele 111-117

### Allgemeine Arbeitsvorschrift:

Zu 0,125 Mol einer Verbindung der Formel (IIIa) mit X = Wasserstoff wurden in 250 ml Eisessig, dem 1,25 ml konzentrierte Salzsäure zugesetzt worden waren, bei Raumtemperatur (22°C) im Verlauf von 2 Stunden 22,4 g (0,14 Mol) Brom gelöst in 50 ml Eisessig zugetropft. Es wurde weitere 2 Stunden bei Raumtemperatur nachgerührt. Dann wurde das Reaktionsgemisch auf 1 l Eiswasser gegossen, die organische Phase abgetrennt, die wäßrige Phase zweimal mit je 100 ml Dichlormethan extrahiert, die vereinigten organischen Phasen zweimal mit je 150 ml Wasser gewaschen und über Magnesiumsulfat getrocknet. Danach wurde im Wasserstrahlvakuum das Lösungsmittel abgezogen. Die Einzelheiten der durchgeführten Umsetzungen sind aus Tabelle 1 ersichtlich, ebenso die durch Aufnahme des ¹H-NMR-Spektrums (in CDCl₃ mit Tetramethylsilan als internen Standard) vorgenommene Charakterisierung der erhaltenen Produkte (angegeben ist jeweils der δ-Wert in ppm für die Protonen der -CH₂-X-Gruppe).

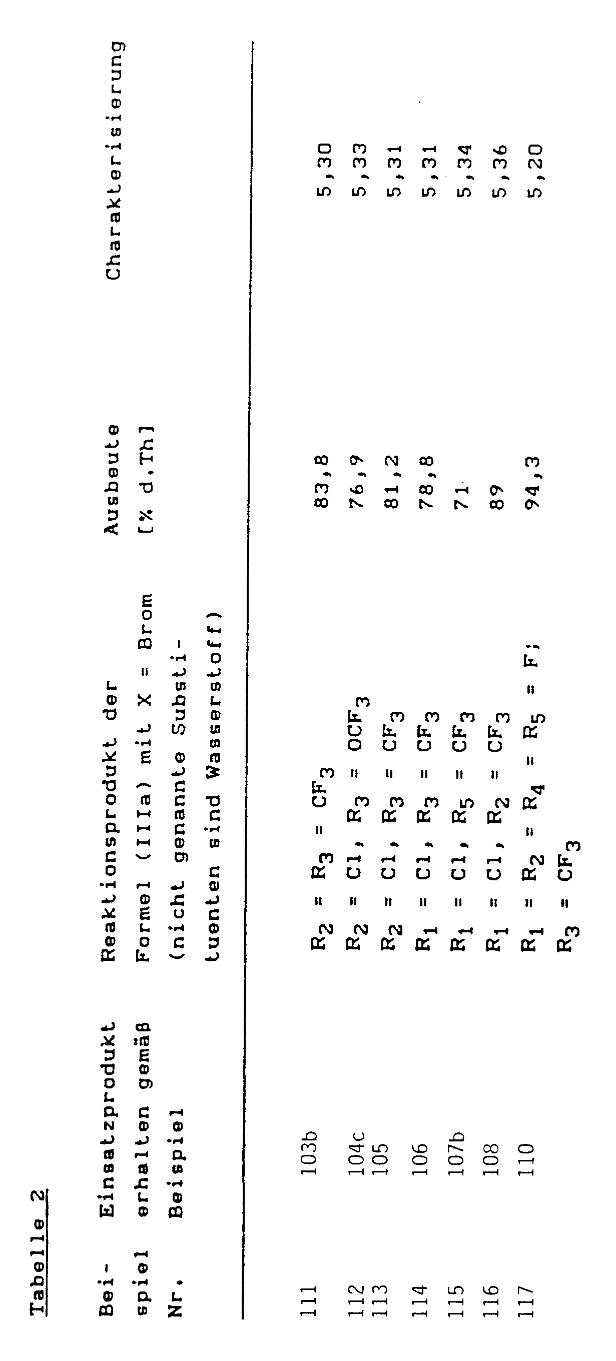

### Beispiel 118

Zu 48 g (0,275 Mol) 2,4,5-Trifluoracetophenon (erhalten gemäß Beispiel 109 gelöst in 400 ml Dichlormethan wurden bei Raumtemperatur (22°C) 40,8 g (0,3 Mol) Sulfurylchlorid getropft und bis zum Ende der Chlorwasserstoffentwicklung (rund 2 Stunden) nachgerührt. Dann wurden dem Reaktionsgemisch 600 ml Wasser zugegeben, die organische Phase abgetrennt, diese mit Natriumhydrogencarbonat-Lösung neutral gewaschen, über Magnesiumsulfat getrocknet und im Wasserstrahlvakuum das Lösungsmittel abgezogen. Es wurden als öliger Rückstand 48,6 g 2,4,5-Trifluorphenacylchlorid erhalten, das entspricht 84,5 % der Theorie. Die wie bei den Beispielen 111 bis 117 vorgenommene Charakterisierung des Produktes lieferte einen δ-Wert von 5.29 ppm.

### Anwendungsbeispiele

In den folgenden Anwendungsbeispielen wurden die nachstehend aufgeführten Verbindungen als Vergleichssubstanzen eingesetzt: 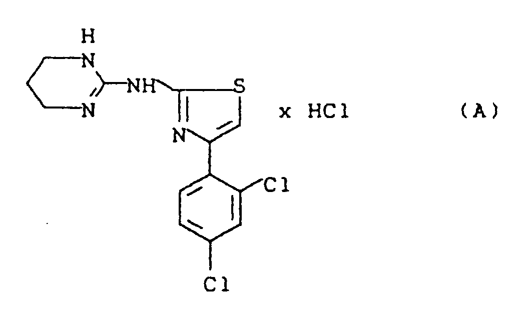
4-(2,4-Dichlorphenyl)-2-[2-(1,4,5,6-tetrahydropyrimidinyl)-amino]-thiazol-Hydrochlorid 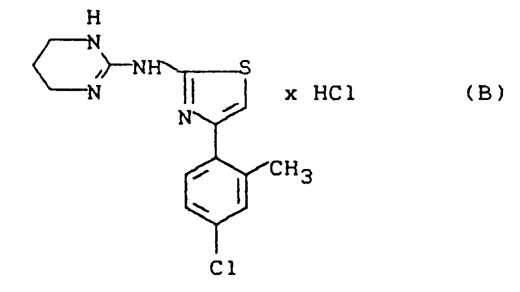
4-(4-Chlor-2-methylphenyl)-2-[2-(1,4,5,6-tetrahydropyrimidinyl)-amino]-thiazol-Hydrochlorid 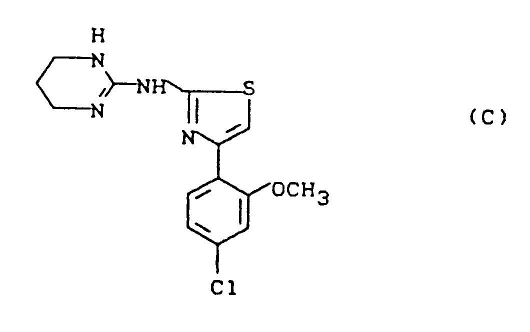
4-(4-Chlor-2-methoxyphenyl)-2-[2-(1,4,5,6-tetrahydropyrimidinyl)-amino]-thiazol
(alle bekannt aus DE-A- 32 20 118)

### Beispiel A

### Antimykotische in-vitro-Wirksamkeit

### Versuchsbeschreibung:

Die in-vitro-Prüfungen wurden mit Keiminokula von durchschnittlich 5 x 10³ Keimen/ml Substrat durchgeführt. Als Nährmedium diente Kimmig-Medium für Hefen und Yeast Nitrogen Base Medium für Schimmelpilze und Dermatophyten.

Die Bebrütungstemperatur betrug 37 °C bei Hefen und 28 °C bei Schimmelpilzen und Dermatophyten, die Bebrütungsdauer lag bei 72 Stunden bei Hefen und 96 bis 120 Stunden bei Dermatophyten und Schimmelpilzen.

Die Beurteilung der Fungizidie erfolgte durch Ausplattieren und erneutes Bebrüten voll gehemmter Ansätze, wobei fungizide Konzentrationen weniger als 100 Keime c. f. u. (colony forming unit) pro ml enthalten.

In diesem Test zeigen die erfindungsgemäßen Verbindungen der Formel (I) gemäß den Herstellungsbeispielen (16), (23), (35), (43), (57), (62), und (72) eine deutlich bessere antimykotische Wirksamkeit als die aus dem Stand der Technik bekannten Vergleichsverbindungen (A), (B) und (C).

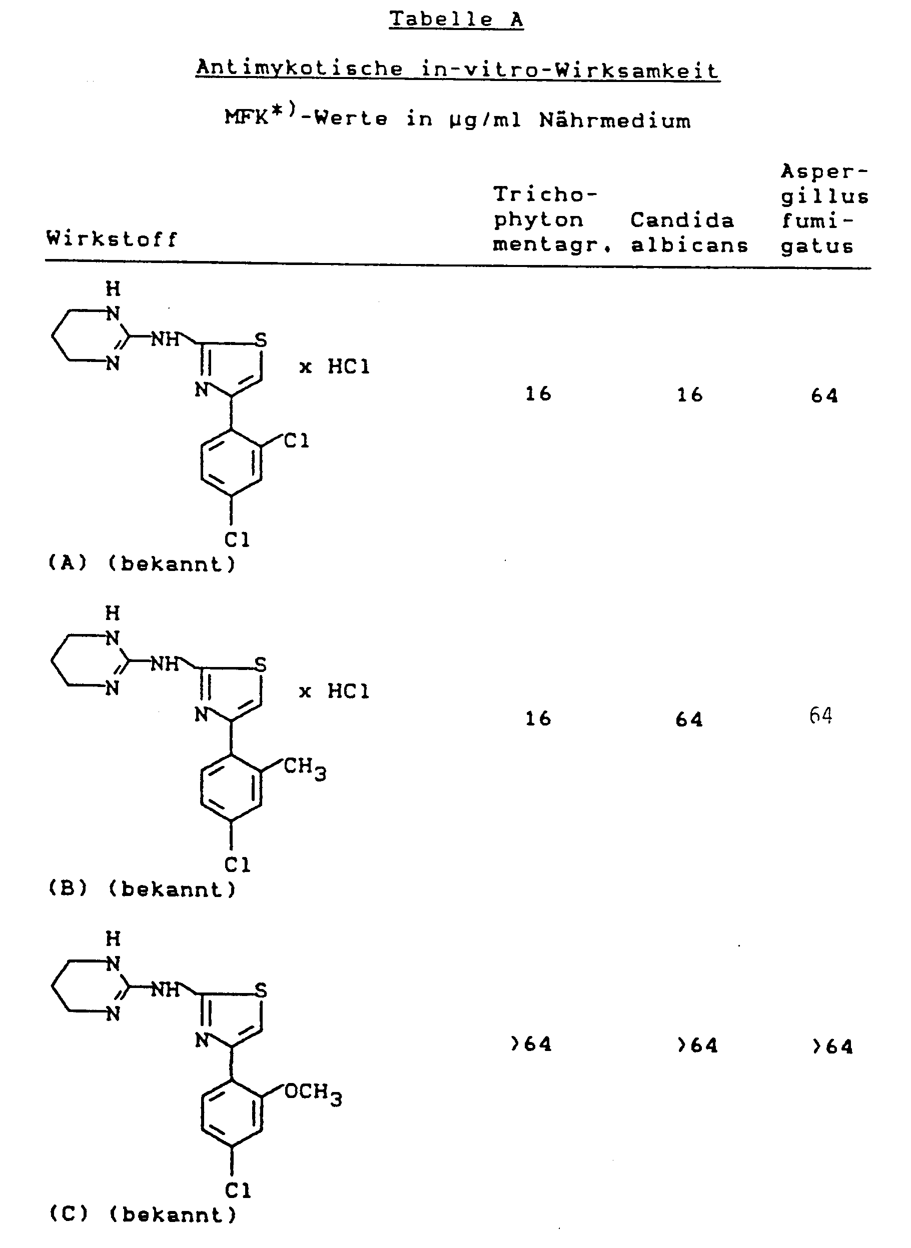

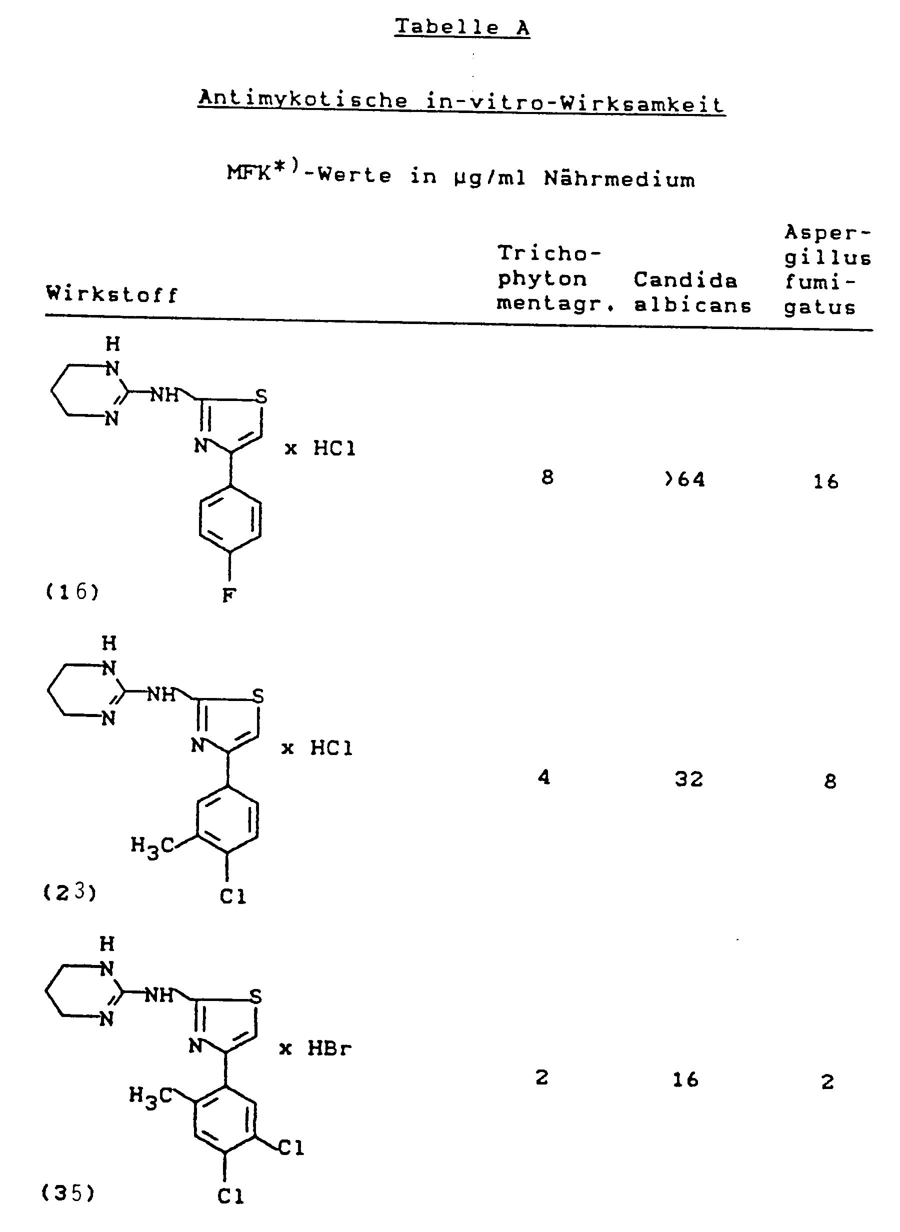

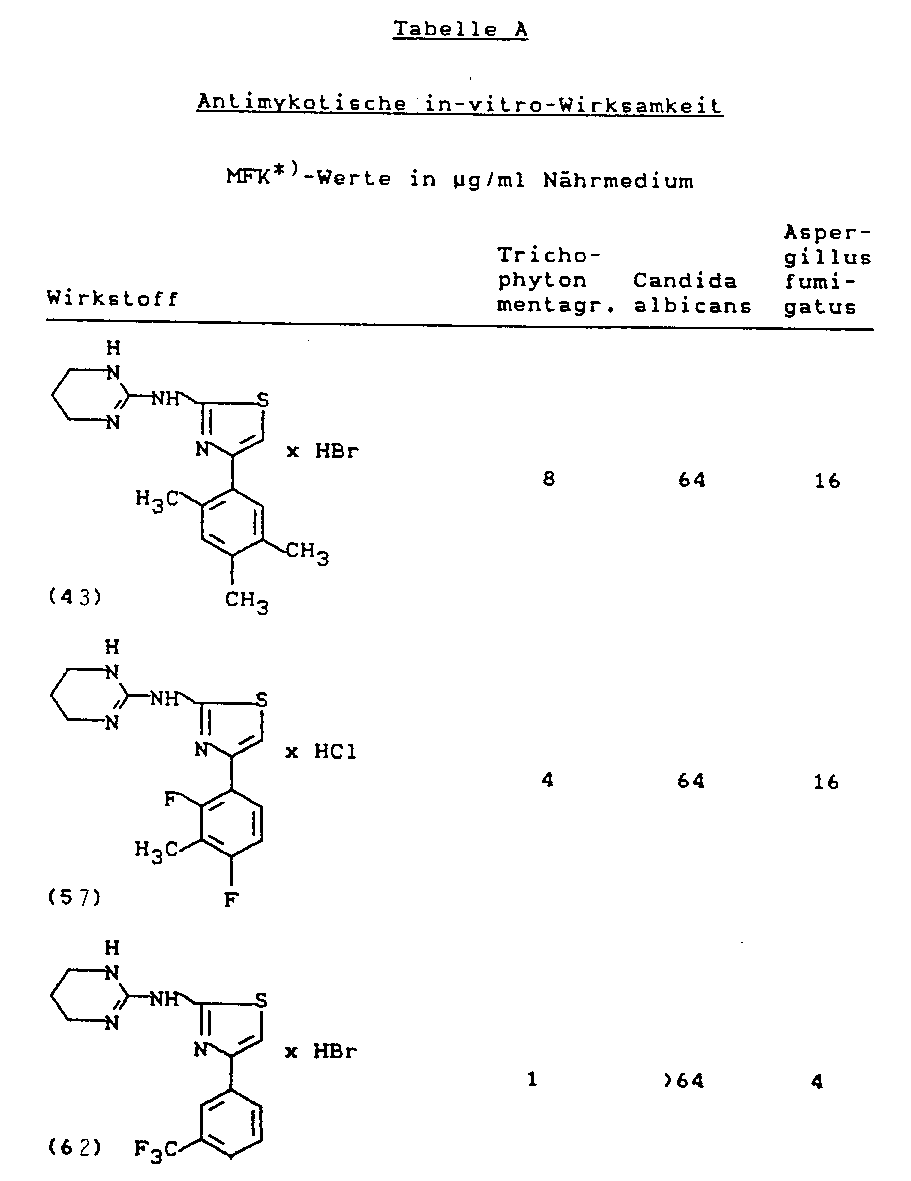

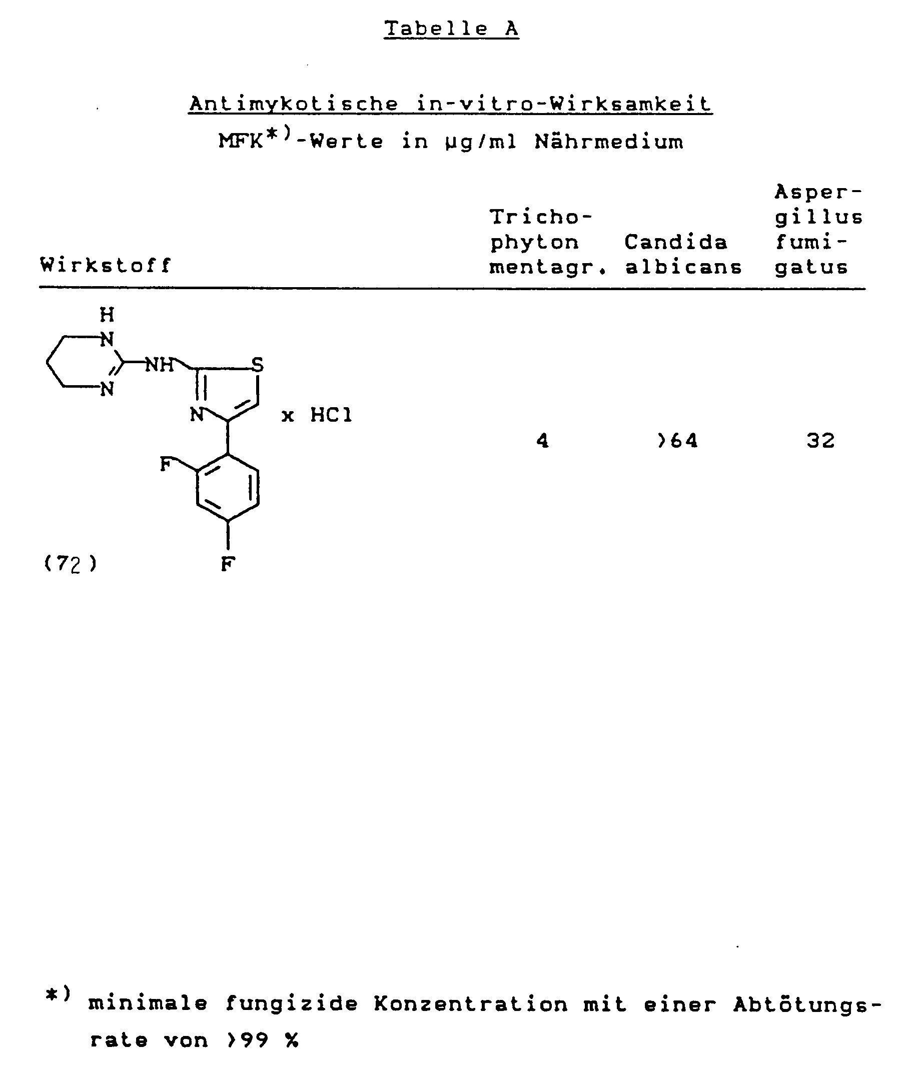

## Patentansprüche (Patentansprüche für folgende(n) Vertragsstaat(en): AT, BE, CH, DE, FR, GB, IT, LI, LU, NL, SE)

1. 2-Aminothiazole der allgemeinen Formel (I) 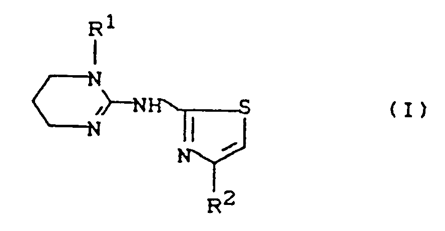 in welcher
R¹ für Wasserstoff oder für geradkettiges oder verzweigtes Alkyl mit 1 bis 4 Kohlenstoffatomen steht und
R² für einfach oder mehrfach, gleich oder verschieden substituiertes Phenyl steht, wobei als Substituenten in Frage kommen: Fluor oder Chlor, geradkettiges oder verzweigtes Alkyl mit 1 bis 6 Kohlenstoffatomen sowie jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Fluor- oder Chloratomen, sowie deren physiologisch verträgliche Säureadditionssalze
- mit Ausnahme der Verbindungen 4-(4-Chlorphenyl)-2-[2-(1,4,5,6-tetrahydropyrimidinyl)amino]-thia zol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydropyrimidinyl)-amino]-thiazol sowie mit Ausnahme der physiologisch verträglichen Säureadditionssalze dieser Verbindungen -.

2. 2-Aminothiazole gemäß Anspruch 1, in denen
R¹ für Wasserstoff oder Methyl steht und
R² für ein-bis fünffach gleich oder verschieden substituiertes Phenyl steht, wobei als Substituenten in Frage kommen: Fluor, Chlor, geradkettiges oder verzweigtes Alkyl mit 1 bis 4 Kohlenstoffatomen oder jeweils geradkettiges oder verzweigtes Halogenalkyl, Halogenalkoxy, Dioxyhalogenalkylen oder Halogenalkylthio mit jeweils 1 bis 3 Kohlenstoffatomen und 1 bis 7 gleichen oder verschiedenen Fluor- oder Chloratomen, sowie deren physiologisch verträgliche Säureadditionssalze
- mit Ausnahme der Verbindungen 4-(4-Chlorphenyl)-2-[2-(1,4,5,6-tetrahydropyrimidinyl)-amino]-thiazol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydro pyrimidinyl)-amino]-thiazol sowie mit Ausnahme der physiologisch verträglichen Säureadditionssalze dieser Verbindungen -.

3. Verfahren zur Herstellung von 2-Aminothiazolen gemäß Ansprüchen 1 und 2, dadurch gekennzeichnet, daß man Thioharnstoff-Derivate der Formel (II) 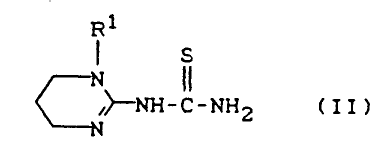 in welcher
R¹ die Bedeutung gemäß Ansprüchen 1 und 2 hat,
mit Keton-Derivaten der Formel (III), 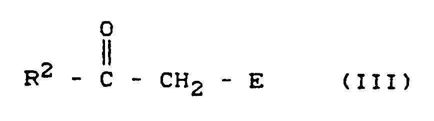 in welcher
R² die Bedeutung gemäß Ansprüchen 1 und 2 hat und
E für Hydroxy oder Halogen steht,
gegebenenfalls in Gegenwart eines Verdünnungsmittels und gegebenenfalls in Gegenwart eines Reaktionshilfsmittels umsetzt und gegebenenfalls anschließend eine Säure addiert.

4. 2-Aminothiazole gemäß Ansprüchen 1 und 2 zur Bekämpfung von Krankheiten.

5. 2-Aminothiazole gemäß Ansprüchen 1 und 2 zur Bekämpfung von Mykosen.

6. 2-Aminothiazole gemäß Ansprüchen 1 und 2 enthaltende Arzneimittel.

7. 2-Aminothiazole gemäß Ansprüchen 1 und 2 enthaltende antimykotische Mittel.

8. Verwendung von 2-Aminothiazolen gemäß Ansprüchen 1 und 2 bei der Herstellung von Arzneimitteln.

## Patentansprüche (Patentansprüche für folgende(n) Vertragsstaat(en): GR)

1. 2-Aminothiazole der allgemeinen Formel (I) 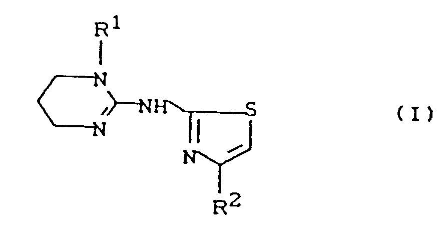 in welcher
R¹ für Wasserstoff oder für geradkettiges oder verzweigtes Alkyl mit 1 bis 4 Kohlenstoffatomen steht und
R² für einfach oder mehrfach, gleich oder verschieden substituiertes Phenyl steht, wobei als Substituenten in Frage kommen: Fluor oder Chlor, geradkettiges oder verzweigtes Alkyl mit 1 bis 6 Kohlenstoffatomen sowie jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Fluor- oder Chloratomen, sowie deren physiologisch verträgliche Säureadditionssalze
- mit Ausnahme der Verbindungen 4-(4-Chlorphenyl)-2-[2-(1,4,5,6-tetrahydropyrimidinyl)amino]-thia zol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydropyrimidinyl)-amino]-thiazol sowie mit Ausnahme der physiologisch verträglichen Säureadditionssalze dieser Verbindungen -.

2. 2-Aminothiazole gemäß Anspruch 1, in denen
R¹ für Wasserstoff oder Methyl steht und
R² für ein-bis fünffach gleich oder verschieden substituiertes Phenyl steht, wobei als Substituenten in Frage kommen: Fluor, Chlor, geradkettiges oder verzweigtes Alkyl mit 1 bis 4 Kohlenstoffatomen oder jeweils geradkettiges oder verzweigtes Halogenalkyl, Halogenalkoxy, Dioxyhalogenalkylen oder Halogenalkylthio mit jeweils 1 bis 3 Kohlenstoffatomen und 1 bis 7 gleichen oder verschiedenen Fluor- oder Chloratomen, sowie deren physiologisch verträgliche Säureadditionssalze
- mit Ausnahme der Verbindungen 4-(4-Chlorphenyl)-2-[2-(1,4,5,6-tetrahydropyrimidinyl)-amino]-thiazol und 4-(2,4-Dichlorphenyl)-2-[2-(1,4,5,6-tetrahydropyrimidinyl)-amino]-thiazol und 4-(4-Chlor-2-methylphenyl)-2-[2-(1,4,5,6-tetrahydro pyrimidinyl)-amino)-thiazol sowie mit Ausnahme der physiologisch verträglichen Säureadditionssalze dieser Verbindungen -.

3. Verfahren zur Herstellung von 2-Aminothiazolen gemäß Ansprüchen 1 und 2, dadurch gekennzeichnet, daß man Thioharnstoff-Derivate der Formel (II) 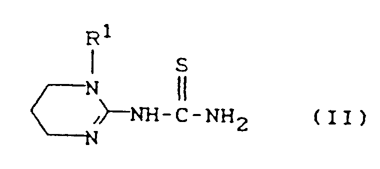 in welcher
R¹ die Bedeutung gemäß Ansprüchen 1 und 2 hat,
mit Keton-Derivaten der Formel (III), 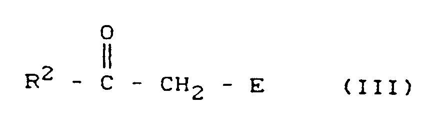 in welcher
R² die Bedeutung gemäß Ansprüchen 1 und 2 hat und
E für Hydroxy oder Halogen steht,
gegebenenfalls in Gegenwart eines Verdünnungsmittels und gegebenenfalls in Gegenwart eines Reaktionshilfsmittels umsetzt und gegebenenfalls anschließend eine Säure addiert.

4. 2-Aminothiazole gemäß Ansprüchen 1 und 2 zur Bekämpfung von Krankheiten.

5. 2-Aminothiazole gemäß Ansprüchen 1 und 2 zur Bekämpfung von Mykosen.

6. Verwendung von 2-Aminothiazolen gemäß Ansprüchen 1 und 2 bei der Herstellung von Arzneimitteln.

## Patentansprüche (Patentansprüche für folgende(n) Vertragsstaat(en): ES)

1. Verfahren zur Herstellung von 2-Aminothiazolen der allgemeinen Formel (I) 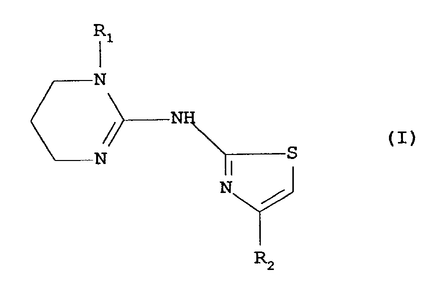 in welcher
R₁ für Wasserstoff oder für geradkettiges oder verzweigtes Alkyl mit 1 bis 4 Kohlenstoffatomen steht und
R₂ für einfach oder mehrfach, gleich oder verschieden substituiertes Phenyl steht, wobei als Substituenten in Frage kommen: Fluor oder Chlor, geradkettiges oder verzweigtes Alkyl mit 1 bis 6 Kohlenstoffatomen sowie jeweils geradkettiges oder verzweigtes Halogenalkyl, Halogenalkoxy, Dioxyhalogenalkylen oder Halogenalkylthio mit jeweils 1 bis 4 Kohlenstoffatomen und 1 bis 9 gleichen oder verschiedenen Fluor- oder Chloratomen, sowie deren physiologisch verträgliche Säureadditionssalze
- mit Ausnahme der Verbindungen 4-(4-Chlorphenyl)-2-[2-(1,4,5,6-tetrahydropyrimidinyl)amino]-thiazol und 4-(2,4-)Dihclorphenyl)-2-[2-(1,4,5,6-tetrahydropyrimidinyl)-amino]-thiazol und 4-(4-Chlor-2-methylphenyl)-2-[2-(1,4,5,6-tetrahydropyrimidinyl)-amino]-thiazol so-wie mit Ausnahme der physiologisch verträglichen Säureadditionssalze dieser Verbindungen -,
dadurch gekennzeichnet, daß man Thioharnstoff-Derivate der Formel (II) 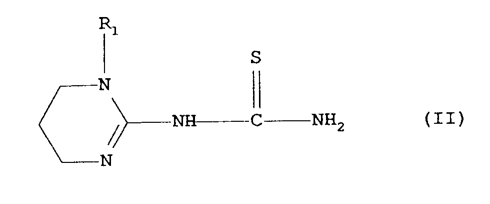 in welcher
R₁ die oben genannte Bedeutung hat
mit Keto-Derivaten der Formel (III) 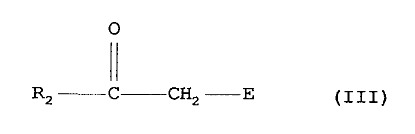 in welcher
R₂ die oben genannte Bedeutung hat und
E für Hydroxy oder Halogen steht,
gegebenenfalls in Gegenwart eines Verdünnungsmittels und gegebenenfalls in Gegenwart eines Reaktionshilfsmittels umsetzt und gegebenenfalls anschließend eine Säure addiert.

## Claims (Claims for the following Contracting State(s): AT, BE, CH, DE, FR, GB, IT, LI, LU, NL, SE)

1. 2-Aminothiazoles of the general formula (I) 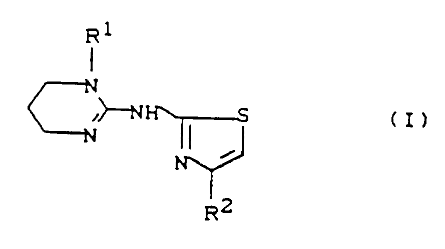 in which
R¹ represents hydrogen or straight-chain or branched alkyl having 1 to 4 carbon atoms and
R² represents phenyl which is monosubstituted or polysubstituted by identical or different substituents, suitable substituents being: fluorine or chlorine, straight-chain or branched alkyl having 1 to 6 carbon atoms and also in each case straight-chain or branched halogenoalkyl, halogenoalkoxy, dioxyhalogenoalkylene or halogenoalkylthio each having 1 to 4 carbon atoms and 1 to 9 identical or different fluorine or chlorine atoms, and their physiologically tolerable acid addition salts,
- with the exception of the compounds 4-(4-chlorophenyl)-2-[2-(1,4,5,6-tetrahydropyrimidinyl)amino]-thiazole and 4-(2,4-dichlorophenyl)-2-[2-(1,4,5,6-tetrahydro pyrimidinyl)-amino]-thiazole and 4-(4-chloro-2-methylphenyl)-2-[2-(1,4,5,6-tetrahydropyrimidinyl)-amino]-thiazole and with the exception of the physiologically tolerable acid addition salts of these compounds.

2. 2-Aminothiazoles according to Claim 1, in which
R¹ represents hydrogen or methyl and
R² represents phenyl which is monosubstituted to pentasubstituted by identical or different substituents, suitable substituents being: fluorine, chlorine, straight-chain or branched alkyl having 1 to 4 carbon atoms or in each case straight-chain or branched halogenoalkyl, halogenoalkoxy, dioxyhalogenoalkylene or halogenoalkylthio each having 1 to 3 carbon atoms and 1 to 7 identical or different fluorine or chlorine atoms, and their physiologically tolerable acid addition salts,
- with the exception of the compounds 4-(4-chlorophenyl)-2-[2-(1,4,5,6-tetrahydropyrimidinyl)-amino]-thiazole and 4-(2,4-dichlorophenyl)-2-[2-(1,4,5,6-tetrahydropyrimidinyl)-amino]-thiazole and 4-(4-chloro-2-methylphenyl)-2-[2-(1,4,5,6-tetrahydropyrimidinyl)-amino]-thiazole and also with the exception of the physiologically tolerable acid addition salts of these compounds.

3. Process for the preparation of 2-aminothiazoles according to Claims 1 and 2, characterized in that thiourea derivatives of the formula (II) 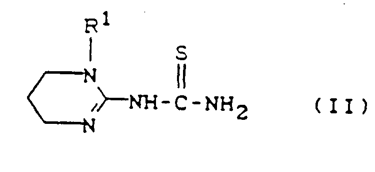 in which
R¹ has the meaning according to Claims 1 and 2,
are reacted with ketone derivatives of the formula (III) 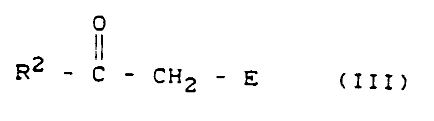 in which
R² has the meaning according to Claims 1 and 2 and
E represents hydroxyl or halogen,
if appropriate in the presence of a diluent and if appropriate in the presence of a reaction auxiliary and, if desired, an acid is then added.

4. 2-Aminothiazoles according to Claims 1 and 2 for combating diseases.

5. 2-Aminothiazoles according to Claims 1 and 2 for combating mycoses.

6. Medicaments containing 2-aminothiazoles according to Claims 1 and 2.

7. Antimycotic agents containing 2-aminothiazoles according to Claims 1 and 2.

8. Use of 2-aminothiazoles according to Claims 1 and 2 for the production of medicaments.

## Claims (Claims for the following Contracting State(s): GR)

1. 2-Aminothiazoles of the general formula (I) 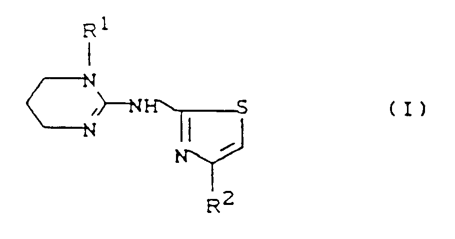 in which
R¹ represents hydrogen or straight-chain or branched alkyl having 1 to 4 carbon atoms and
R² represents phenyl which is monosubstituted or polysubstituted by identical or different substituents, suitable substituents being: fluorine or chlorine, straight-chain or branched alkyl having 1 to 6 carbon atoms and also in each case straight-chain or branched halogenoalkyl, halogenoalkoxy, dioxyhalogenoalkylene or halogenoalkylthio each having 1 to 4 carbon atoms and 1 to 9 identical or different fluorine or chlorine atoms, and their physiologically tolerable acid addition salts,
- with the exception of the compounds 4-(4-chlorophenyl)-2-[2-(1,4,5,6-tetrahydropyrimidinyl)amino]-thiazole and 4-(2,4-dichlorophenyl)-2-[2-(1,4,5,6-tetrahydro pyrimidinyl)-amino]-thiazole and 4-(4-chloro-2-methylphenyl)-2-[2-(1,4,5,6-tetrahydropyrimidinyl)-amino]-thiazole and with the exception of the physiologically tolerable acid addition salts of these compounds.

2. 2-Aminothiazoles according to Claim 1, in which
R¹ represents hydrogen or methyl and
R² represents phenyl which is monosubstituted to pentasubstituted by identical or different substituents, suitable substituents being: fluorine, chlorine, straight-chain or branched alkyl having 1 to 4 carbon atoms or in each case straight-chain or branched halogenoalkyl, halogenoalkoxy, dioxyhalogenoalkylene or halogenoalkylthio each having 1 to 3 carbon atoms and 1 to 7 identical or different fluorine or chlorine atoms, and their physiologically tolerable acid addition salts,
- with the exception of the compounds 4-(4-chlorophenyl)-2-[2-(1,4,5,6-tetrahydropyrimidinyl)-amino]-thiazole and 4-(2,4-dichlorophenyl)-2-[2-(1,4,5,6-tetrahydropyrimidinyl)-amino]-thiazole and 4-(4-chloro-2-methylphenyl)-2-[2-(1,4,5,6-tetrahydropyrimidinyl)-amino]-thiazole and also with the exception of the physiologically tolerable acid addition salts of these compounds.

3. Process for the preparation of 2-aminothiazoles according to Claims 1 and 2, characterized in that thiourea derivatives of the formula (II) 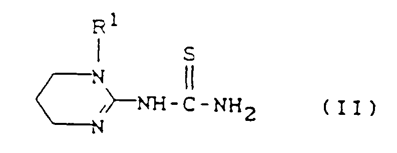 in which
R¹ has the meaning according to Claims 1 and 2,
are reacted with ketone derivatives of the formula (III) 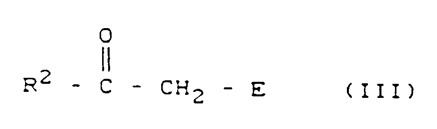 in which
R² has the meaning according to Claims 1 and 2 and
E represents hydroxyl or halogen,
if appropriate in the presence of a diluent and if appropriate in the presence of a reaction auxiliary and, if desired, an acid is then added.

4. 2-Aminothiazoles according to Claims 1 and 2 for combating diseases.

5. 2-Aminothiazoles according to Claims 1 and 2 for combating mycoses.

6. Use of 2-aminothiazoles according to Claims 1 and 2 for the production of medicaments.

## Claims (Claims for the following Contracting State(s): ES)

1. Process for the preparation of 2-aminothiazoles of the general formula (I) 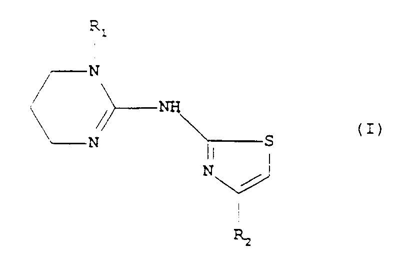 in which
R¹ represents hydrogen or straight-chain or branched alkyl having 1 to 4 carbon atoms and
R² represents phenyl which is monosubstituted or polysubstituted by identical or different substituents, suitable substituents being: fluorine or chlorine, straight-chain or branched alkyl having 1 to 6 carbon atoms and also in each case straight-chain or branched halogenoalkyl, halogenoalkoxy, dioxyhalogenoalkylene or halogenoalkylthio each having 1 to 4 carbon atoms and 1 to 9 identical or different fluorine or chlorine atoms, and their physiologically tolerable acid addition salts,
- with the exception of the compounds 4-(4-chlorophenyl)-2-[2-(1,4,5,6-tetrahydropyrimidinyl)amino]-thiazole and 4-(2,4-dichlorophenyl)-2-[2-(1,4,5,6-tetrahydro pyrimidinyl)-amino]-thiazole and 4-(4-chloro-2-methylphenyl)-2-[2-(1,4,5,6-tetrahydropyrimidinyl)-amino]-thiazole and with the exception of the physiologically tolerable acid addition salts of these compounds,
characterized in that thiourea derivatives of the formula (II) 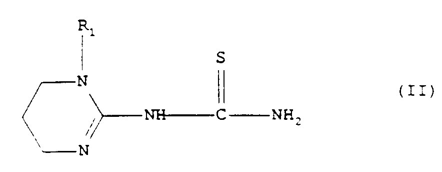 in which
R¹ has the meaning according to Claims 1 and 2,
are reacted with ketone derivatives of the formula (III) 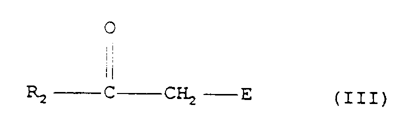 in which
R² has the abovementioned meaning and
E represents hydroxyl or halogen,
if appropriate in the presence of a diluent and if appropriate in the presence of a reaction auxiliary and, if desired, an acid is then added.

## Revendications (Revendications pour l'(les) Etat(s) contractant(s) suivant(s): AT, BE, CH, DE, FR, GB, IT, LI, LU, NL, SE)

1. 2-aminothiazoles de formule générale (I) 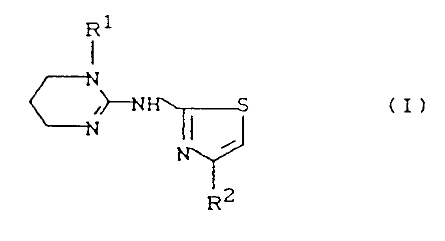 dans laquelle
R¹ représente l'hydrogène ou un groupe alkyle à chaîne droite ou ramifiée ayant 1 à 4 atomes de carbone et
R² est un groupe phényle portant un ou plusieurs substituants identiques ou différents, en considérant comme substituants : le fluor ou le chlore, un groupe alkyle à chaîne droite ou ramifiée ayant 1 à 6 atomes de carbone ainsi qu'un groupe halogénalkyle, halogénalkoxy, dioxyhalogénalkylène ou halogénalkylthio chacun à chaîne droite ou ramifiée, avec chacun 1 à 4 atomes de carbone et 1 à 9 atomes de fluor ou de chlore identiques ou différents, ainsi que leurs sels d'addition d'acides acceptables du point de vue physiologique
- à l'exception des composés 4-(4-chlorphényl)-2-[2-(1,4,5,6-tétrahydropyrimidinyl)amino]-thiazole, 4-(2,4-dichlorophényl)-2-[2-(1,4,5,6-tétrahydropyrimidinyl)-amino]-thiazole et 4-(4-chloro-2-méthylphényl)-2-[2-(1,4,5,6-tétrahydropyrimidinyl)-amino]-thiazole de même qu'à l'exception des sels d'addition d'acides acceptables du point de vue physiologique de ces composés -.

2. 2-aminothiazoles suivant la revendication 1, dans lesquels
R¹ est l'hydrogène ou le groupe méthyle et
R² est un groupe phényle portant 1 à 5 substituants identiques ou différents, en considérant comme substituants : le fluor ou le chlore, un groupe alkyle à chaîne droite ou ramifiée ayant 1 à 4 atomes de carbone ou un groupe halogénalkyle, halogénalkoxy, dioxyhalogénalkylène ou halogénalkylthio chacun à chaîne droite ou ramifiée, avec chacun 1 à 3 atomes de carbone et 1 à 7 atomes de fluor ou de chlore identiques ou différents, ainsi que leurs sels d'addition d'acides acceptables du point de vue physiologique,
- à l'exception des composés 4-(4-chlorophényl)-2-[2-(1,4,5,6-tétrahydropyrimidinyl)amino]-thiazole, 4-(2,4-dichlorophényl)-2-[2-(1,4,5,6-tétrahydropyrimidinyl)-amino]-thiazole et 4-(4-chloro-2-méthylphényl)-2-[2-(1,4,5,6-tétrahydropyrimidinyl)-amino]-thiazole de même qu'à l'exception des sels d'addition d'acides physiologiquement acceptables de ces composés -.

3. Procédé de production de 2-aminothiazoles suivant les revendications 1 et 2, caractérisé en ce qu'on fait réagir des dérivés de thiourée de formule (II) 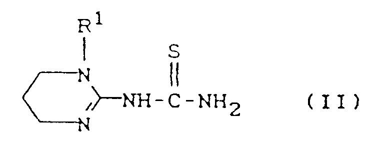 dans laquelle
R¹ a la définition indiquée dans les revendications 1 et 2,
avec des dérivés cétoniques de formule (III), 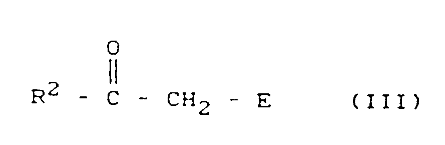 dans laquelle
R² a la définition indiquée dans les revendications 1 et 2 et
E est un groupe hydroxy ou un halogène,
le cas échéant, en présence d'un diluant et en la présence éventuelle d'une substance auxiliaire de réaction et on additionne ensuite éventuellement un acide.

4. 2-aminothiazoles suivant les revendications 1 et 2, destinés à combattre des maladies.

5. 2-aminothiazoles suivant les revendications 1 et 2, destinés à combattre des mycoses.

6. Médicaments contenant des 2-aminothiazoles suivant les revendications 1 et 2.

7. Compositions antimycosiques contenant des 2-aminothiazoles suivant les revendications 1 et 2.

8. Utilisation de 2-aminothiazoles suivant les revendications 1 et 2 dans la préparation de médicaments.

## Revendications (Revendications pour l'(les) Etat(s) contractant(s) suivant(s): GR)

1. 2-aminothiazoles de formule générale (I) 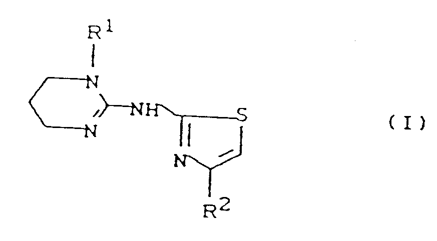 dans laquelle
R¹ représente l'hydrogène ou un groupe alkyle à chaîne droite ou ramifiée ayant 1 à 4 atomes de carbone et
R² est un groupe phényle portant un ou plusieurs substituants identiques ou différents, en considérant comme substituants : le fluor ou le chlore, un groupe alkyle à chaîne droite ou ramifiée ayant 1 à 6 atomes de carbone ainsi qu'un groupe halogénalkyle, halogénalkoxy, dihalogénalkylène ou halogénalkylthio chacun à chaîne droite ou ramifiée, avec chacun 1 à 4 atomes de carbone et 1 à 9 atomes de fluor ou de chlore identiques ou différents, ainsi que leurs sels d'addition d'acides acceptables du point de vue physiologique
- à l'exception des composés 4-(4-chlorphényl)-2-[2-(1,4,5,6-tétrahydropyrimidinyl)amino]-thiazole, 4-(2,4-dichlorophényl)-2-[2-(1,4,5,6-tétrahydropyrimidinyl)-amino]-thiazole et 4-(4-chloro-2-méthylphényl)-2-[2-(1,4,5,6-tétrahydropyrimidinyl)-amino]-thiazole de même qu'à l'exception des sels d'addition d'acides acceptables du point de vue physiologique de ces composés -.

2. 2-aminothiazoles suivant la revendication 1, dans lesquels
R¹ est l'hydrogène ou le groupe méthyle et
R² est un groupe phényle portant 1 à 5 substituants identiques ou différents, en considérant comme substituants : le fluor ou le chlore, un groupe alkyle à chaîne droite ou ramifiée ayant 1 à 4 atomes de carbone ou un groupe halogénalkyle, halogénalkoxy, dioxyhalogénalkylène ou halogénalkylthio chacun à chaîne droite ou ramifiée, avec chacun 1 à 3 atomes de carbone et 1 à 7 atomes de fluor ou de chlore identiques ou différents, ainsi que leurs sels d'addition d'acides acceptables du point de vue physiologique,
- à l'exception des composés 4-(4-chlorophényl)-2-[2-(1,4,5,6-tétrahydropyrimidinyl)amino]-thiazole, 4-(2,4-dichlorophényl)-2-[2-(1,4,5,6-tétrahydropyrimidinyl)-amino]-thiazole et 4-(4-chloro-2-méthylphényl)-2-[2-(1,4,5,6-tétrahydropyrimidinyl)-amino]-thiazole de même qu'à l'exception des sels d'addition d'acides physiologiquement acceptables de ces composés -.

3. Procédé de production de 2-aminothiazoles suivant les revendications 1 et 2, caractérisé en ce qu'on fait réagir des dérivés de thiourée de formule (II) 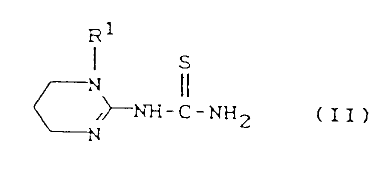 dans laquelle
R¹ a la définition indiquée dans les revendications 1 et 2,
avec des dérivés cétoniques de formule (III), 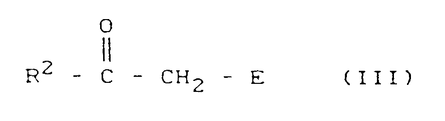 dans laquelle
R² a la définition indiquée dans les revendications 1 et 2 et
E est un groupe hydroxy ou un halogène,
le cas échéant, en présence d'un diluant et en la présence éventuelle d'une substance auxiliaire de réaction et on additionne ensuite éventuellement un acide.

4. 2-aminothiazoles suivant les revendications 1 et 2, destinés à combattre des maladies.

5. 2-aminothiazoles suivant les revendications 1 et 2, destinés à combattre des mycoses.

6. Utilisation des 2-aminothiazoles suivant les revendications 1 et 2 pour la préparation de médicaments.

## Revendications (Revendications pour l'(les) Etat(s) contractant(s) suivant(s): ES)

1. Procédé de production de 2-aminothiazoles de formule générale (I) 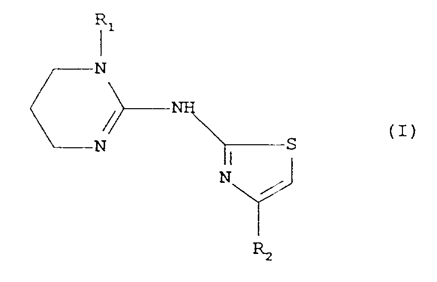 dans laquelle
R₁ représente l'hydrogène ou un groupe alkyle à chaîne droite ou ramifiée ayant 1 à 4 atomes de carbone et
R₂ est un groupe phényle portant un ou plusieurs substituants identiques ou différents, en considérant comme substituants : le fluor ou le chlore, un groupe alkyle à chaîne droite ou ramifiée ayant 1 à 6 atomes de carbone ainsi qu'un groupe halogénalkyle, halogénalkoxy, dioxyhalogénalkylène ou halogénalkylthio chacun à chaîne droite ou ramifiée, avec chacun 1 à 4 atomes de carbone et 1 à 9 atomes de fluor ou de chlore identiques ou différents, ainsi que leurs sels d'addition d'acides acceptables du point de vue physiologique
- à l'exception des composés 4-(4-chlorphényl)-2-[2-(1,4,5,6-tétrahydropyrimidinyl)amino]-thiazole, 4-(2,4-dichlorophényl)-2-[2-(1,4,5,6-tétrahydropyrimidinyl)-amino]-thiazole et 4-(4-chloro-2-méthylphényl)-2-[2-(1,4,5,6-tétrahydropyrimidinyl)-amino]-thiazole de même qu'à l'exception des sels d'addition d'acides acceptables du point de vue physiologique de ces composés -,
caractérisé en ce qu'on fait réagir des dérivés de thiourée de formule (II) 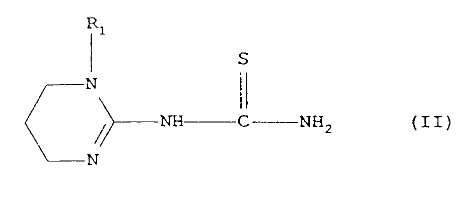 dans laquelle
R₁ a la définition indiquée ci-dessus
avec des dérivés cétoniques de formule (III) 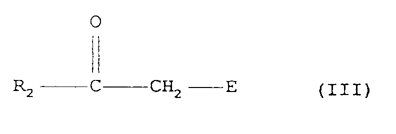 dans laquelle
R₂ a la définition indiquée ci-dessus et
E est un groupe hydroxy ou un halogène,
le cas échéant en présence d'un diluant et en la présence éventuelle d'une substance auxiliaire de réaction et on additionne ensuite, le cas échéant, un acide.
